# EUROPÄISCHE PATENTSCHRIFT

(11) **EP 2 464 361 B1**
(45) Veröffentlichungstag und Bekanntmachung des Hinweises auf die Patenterteilung: **17.01.2018**
(21) Anmeldenummer: 10763304.2
(22) Anmeldetag: 11.08.2010
(51) Int. Cl.: A61P 29/00, A61K 35/747, A61P 37/00, A23L 33/135

(54) **ZUSAMMENSETZUNG MIT STÄMMEN VON LACTOBAZILLUS FERMENTUM ZUR VERWENDUNG ZUR VERSTÄRKUNG DER IMMUNABWEHR**
COMPOSITION HAVING STRAINS OF LACTOBACILLUS FERMENTUM FOR USE BOOSTING THE IMMUNE SYSTEM
COMPOSITION RENFERMANT DES SOUCHES DE LACTOBACILLUS FERMENTUM POUR UTLISATION À RENFORCER LE SYSTÈME IMMUNITAIRE

(30) Priorität: 11.08.2009 DE 102009037089
(43) Veröffentlichungstag der Anmeldung: 20.06.2012
(73) Patentinhaber: Schrezenmeir, Jürgen, 76131 Karlsruhe (DE); Heller, Knut J., 24109 Kiel (DE)
(72) Erfinder: Schrezenmeir, Jürgen, 76131 Karlsruhe (DE); Heller, Knut J., 24109 Kiel (DE)
(74) Vertreter: Pöhner, Wilfried Anton
(86) Internationale Anmeldenummer: PCT/DE2010/000953
(87) Internationale Veröffentlichungsnummer: WO 2011/018080

(56) Entgegenhaltungen:
- WO-A1-02/34273
- WO-A1-2008/060198
- WO-A1-2008/079009
- WO-A2-03/022255
- US-A1- 2006 083 723
- DATABASE WPI Week 200841 Thomson Scientific, London, GB; AN 2008-G54949 XP002619894, & WO 2008/064521 A1 (PHARMAPEP RES & DEV SHENZHEN CO LTD) 5. Juni 2008 (2008-06-05)
- DATABASE WPI Week 200482 Thomson Scientific, London, GB; AN 2004-830133 XP002619909, & KR 2004 068 820 A (PL BIO CO LTD) 2. August 2004 (2004-08-02)

## Beschreibung

Die Erfindung bezieht sich auf eine pharmazeutische und/oder dietätische Zusammensetzung zur Verwendung zur Verstärkung der menschlichen Immunabwehr.

Die Immunabwehr ist ein allzeit präsentes und aktives, biologisches Abwehrsystem höherer Lebewesen, das Gewebeschädigungen durch Krankheitserreger verhindert. Es besteht aus verschiedenen Organen, sowie Zellen und Molekülen der verschiedensten Typen, die ein komplexes Netzwerk bilden. Es entfernt in den Körper eingedrungene Mikroorganismen und andere, fremde Substanzen und ist außerdem in der Lage, fehlerhaft gewordene körpereigene Zellen zu zerstören. Die Immunabwehr hat eine große Bedeutung für die körperliche Unversehrtheit von Lebewesen, denn praktisch alle Organismen sind ständig den Einflüssen der belebten Umwelt ausgesetzt, von denen manche sogar eine existentielle Bedrohung sind.

Wenn schädliche Mikroorganismen in den Körper eindringen, kann dies zu Funktionsstörungen und Krankheiten führen. Typische Krankheitserreger sind Bakterien, Viren und Pilze sowie einzellige Parasiten, z. B. Protozoen oder andere Plasmodien, oder mehrzellige Parasiten wie z. B. Bandwürmer.

Derzeit wachsen zunehmend häufiger neue Arten von schädlichen Bakterien und anderen unwillkommenen Mikroorganismen heran, die nicht nur gegen eines sondern oft sogar gegen mehrere Arzneimittel resistent sind, die gegen die bisher bekannten Arten noch sehr wirkungsvoll waren. Ein bedrohliches Beispiel ist im Juli 2009 der Erreger N1H1; auch Schweinegrippe genannt, dessen Mutanten zu einer Pandemie führen könnten. Deshalb besteht aktuell ein verstärktes Interesse an einer Verstärkung und Erhöhung der Wirksamkeit der körpereigenen Abwehr.

Interesse an einer Verstärkung und Erhöhung der Wirksamkeit der körpereigenen Abwehr.

Dafür sind Wirkstoffe mit einer immunmodulierenden Wirkung sinnvoll. Die immunmodulierende Wirkung kann unterschieden werden in eine sog. natürliche Immunität (natural oder innate immunity), die bereits ein Neugeborenes mitbringt und die adaptive oder erworbene Immunität, die sich erst im Laufe des Lebens durch den Kontakt mit verschiedenen Eindringlingen ausbildet. Zur adaptiven Immunität gehören die T-Helfer-Zellen, die ein wesentlicher Teil des lernfähigen Abwehrsystems des Körpers sind. Aus der Gruppe der T-Helfer-Zellen sind die T-Helfer1 und die T-Helfer2-Zellen besonders wichtig.

Bei Krankheiten ist es eine bekannte und effiziente Behandlung, die sog. Th1/Th2-Antwort so zu beeinflussen, dass die jeweilige Krankheit sehr viel schneller und umfangreicher bekämpft wird. Dazu wird ihre Wirkung durch Gabe von stimulierenden Substanzen verstärkt.

So nennt z.B. die Patentanmeldung KR 1007 42900 den Lactobacillus rhamnosus IDCC 3201 als einen Bakterienstamm, der als probiotischer Zusatz Nahrungsmitteln hinzugefügt wird, um atopischer Dermatitis entgegen zu wirken und ihr vorzubeugen. Laborversuche legen nahe, dass dieser Bakterienstamm bei der Linderung und Vorbeugung von allergischen Reaktionen wirkt, indem er die Balance von T-Helfer1 und T-Helfer2-Zellen aufrechterhält.

Der Fundort dieses Bakterienstammes in den Fäkalien von koreanischen Kindern sollte bei einer dem Stand der Technik entsprechenden Aufbereitung eigentlich kein Nachteil sein, erweckt jedoch nicht von vornherein ausschließlich positive Erwartungen.

In jedem Fall ist von diesem Bakterienstamm jedoch keine Wirkung zu erwarten, wenn es um eine andere, zielgerichtete Verschiebung des T-Helfer1 zu T-Helfer2-Verhältnisses geht, die auf andere Krankheiten wirkt.

Auf diesem Hintergrund hat sich die Erfindung die Aufgabe gestellt, Wirkstoffe aus einer Gruppe zu finden, die immunmodulierend wirken, indem sie die Immunantworten des Körpers verstärken und dabei entweder die natürliche Immunität verstärken oder die adaptive Immunität unterstützen, indem sie T-Helferzellen möglichst direkt zu einer verstärkten Aktivität anregt und dabei die Wirkung gezielt und vorrangig entweder auf die T-Helfer-Zellen 1 oder auf die T-Helfer-Zellen 2 lenkt. Die Erfindung ist die, wie in den Ansprüchen definiert. Als Lösung präsentiert die Erfindung eine pharmazeutische und/oder dietätische Zusammensetzung, in der zur Verstärkung der adaptiven Immunabwehr mittels T-Helfer1- und T-Helfer2-Zellen Bakterien der Art Lactobacillus fermentum von wenigstens einem der Stämme K1-Lb1 oder K1-Lb6 oder K2-Lb4 oder K6-Lb4 oder K7-Lb1 oder K8-Lb1 oder K9-Lb3 und/oder der Art Lactobacillus plantarum vom Stamm K4-Lb6 und/oder zur Verstärkung der nativen Immunabwehr Bakterien der Art Lactobacillus fermentum von wenigstens einem der Stämme K2-Lb6 oder K11-Lb3 enthalten sind.

Die hier benutzten Bezeichnungen KxLBy sind aus der Systematik der ursprünglichen Untersuchung von Kimere entstanden. K steht für Kimere, der Quelle der hier vorgestellten bisher unbekannten Stämme, Lb steht für Lactobacillus. Aus der großen Anzahl von untersuchten Spezies wurden einige ausgewählt, so dass der erste Parameter der Bezeichnung- Kx - und der zweite Parameter der Bezeichnung - Lby - keine durchlaufenden Zahlen enthält.

Die Bezeichnungen KxLBy der hier präsentierten Stämme der Art Lactobacillus fermentum führen auf zwei alternativen Arten zu einer eindeutigen Identifikation des jeweiligen Bakterienstammes:
Die erste Alternative ist die bekannte Methode der Hinterlegung eines Abkömmlings der jeweiligen Art bei der DSMZ, der Deutschen Sammlung von Mikroorganismen und Zellkulturen GmbH in der Inhoffenstraße 7B in D-38124 Braunschweig. Nach der für eine Patentanmeldung vorgeschriebenen Prozedur wurden unversehrte und lebende Exemplare der hier genannten Bakterienstämme bei der DSMZ hinterlegt und zwar am 6.August 2009 unter den folgenden Zeichen:
   Lactobacillus fermentum K1-Lb1
   Lactobacillus fermentum K2-Lb6
   Lactobacillus fermentum K7-Lb1
   Lactobacillus fermentum K8-Lb1
   Lactobacillus fermentum K11-Lb3 und
   Lactobacillus plantarum K4-Lb6

Die übrigen Stämme wurden bislang noch nicht bei der DSMZ hinterlegt. Zu ihrer Offenbarung dient die zweite, alternative Methode der Bekanntgabe des PFGE-Bandenmusters.

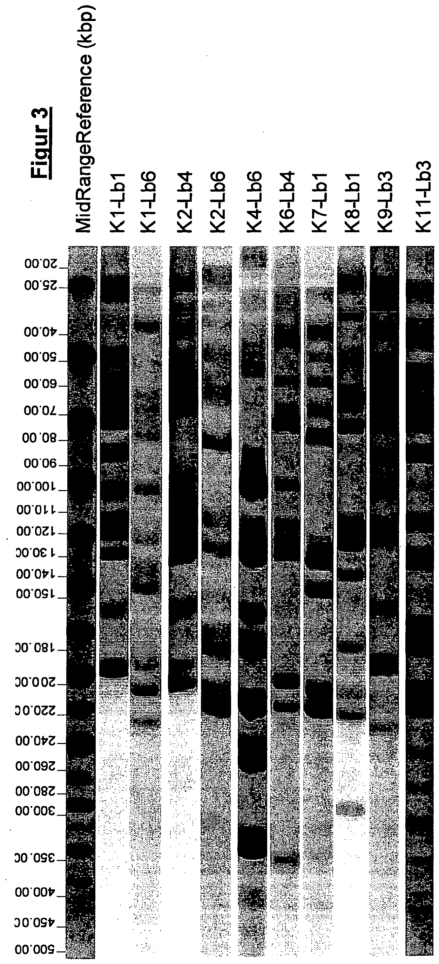

Eine Alternativ zu der Hinterlegung von lebenden Bakterien ist die eindeutige Identifikation mittels Pulsfeldgel-elektrophoresemuster (PFGE-Muster). Für jeden der hier genannten Bakterienstämme sind in der beiliegenden Figur 3 die jeweiligen sogenannten Bandenmuster hinterlegt. Bakterien, die nach der PFGE-Methode analysiert werden und dabei ein gleiches Bandenmuster zeigen, sind damit eindeutig als dieser Stamm der jeweiligen Art identifiziert.

Das Verfahren der Typisierung von Mikroorganismen durch PFGE ist seit 1984 bekannt und zählt zum anerkannten Stand der Technik. Die Forscher Schwartz und Cantor beobachteten, dass beim Anlegen elektrischer Impulse, die in einem bestimmten Winkel im Verhältnis zum Argachosegel periodisch ihre Orientierung änderten, große intakte DNA-Moleküle als Bandenmuster aufgelöst wurden. Dieses Verfahren basiert auf dem US-Patent 4,473,452 und umfasst vier wesentliche Schritte, nämlich
1. Aufbereiten der Proben durch Kultur der Mikroorganismen in einer Nährboullion, Einbetten der Zellen in Gele und das Erhalten immobilisierter, deproteinisierter, intakter DNA-Moleküle.
2. Festlegung des Ablaufs der Elektrophorese für eine optimale Auftrennung zwischen den Molekülen.
3. Ladung der Proben in die Gele und Durchführung der Pulsfeld-Gelelektrophorese mit dem Ergebnis jeweils eines typischen Bandenmusters.
4. Analysieren der Bandenmuster durch Vergleich mit den Bandenmustern ähnlicher Arten von Mikroorganismen. Es zählt zum allgemein akzeptierten Stand der Technik, dass das dabei erhaltene Bandenmuster eine eindeutige Identifikation der jeweiligen Art ermöglicht, etwa vergleichbar mit einem Fingerabdruck einer Person.

Das PFGE-Verfahren ist jedoch nicht der eigentliche Inhalt dieser Patentanmeldung sondern nur eines von zwei benutzten Methoden zur eindeutigen Identifizierung der zu schützenden Bakterienstämme der Art Lactobacillus fermentum. Das allen Arten gemeinsame, erfinderische Merkmal ist die stimulierende Wirkung auf die T-Helfer-Zellen.

Die T-Helfer-Zellen gehören zu den Lymphozyten, eine Untergruppe der "weißen Blutkörperchen", die als die wichtigsten "Agenten" der menschlichen Immunabwehr wirken. Ein erwachsener Mensch hat knapp über tausend unterschiedliche lymphatische Zellen, die etwa zwei Prozent seines Körpergewichts ausmachen. Die Lymphozyten stammen wie alle Blutzellen aus dem Knochenmark und müssen vor ihrem Heranreifen zu funktionierenden Zellen der Immunabwehr noch zusätzliche Entwicklungs- und Differenzierungsstadien durchlaufen.

Ein Teil dieser Vorläuferzellen wandert aus den blutbildenden Geweben direkt in die Thymusdrüse ein und entwickelt sich zu den sogenannten T-Lymphozyten. Sie lernen dort unter anderem, zwischen körpereigenen Zellen und "Eindringlingen" zu unterscheiden. Die restlichen Lymphozyten reifen im Knochenmark (bone marrow) zu den sogenannten B-Zellen heran.

Die wichtigste Eigenschaft der Lymphozyten ist ihre Fähigkeit, nach dem Schlüssel-Schloss-Prinzip mit einer bestimmten Molekülform zu reagieren. Dazu tragen sie als "Schloss" auf ihrer Oberfläche Rezeptoren, die speziell an die Proteinstruktur eines von Millionen von verschiedenen Fremdkörpern angepasst sind - den "Schlüssel". Sie erkennen über die Struktur aus zelleigenem und zellfremdem Protein auf der Oberfläche der Makrophagen einen "neuen Eindringling" und setzen die spezifische Immunabwehr in Gang.

Zur eigentlichen Exekution der Immunabwehr entstehen T-Killerzellen, die sich gezielt mit dem Eindringling verbinden und ihn zerstören. Die Verstärkung dieser menschlichen Immunabwehr durch die erfinderischen Wirkstoffe erfolgt indirekt, nämlich durch die verstärkte Bildung von T-Helferzellen, die B-Lymphozyten dazu anregen, sich in Plasmazellen, die "Antikörperfabriken" des Körpers, zu verwandeln. Diese können im Laufe ihrer kurzen Lebenszeit von nur wenigen Tagen pro Sekunde Tausende spezialisierter Abwehrmoleküle ausschütten.

An dieser Stelle setzt also die Wirkung der hier präsentierten Bakterienstämme ein, indem sie auf eine der beiden Typen der T-Helfer-Zellen einwirken, wobei sie entweder die Anzahl der jeweils entstehenden T-Helfer1 oder T-Helfer2-Zellen erhöhen, wodurch sich zwangsläufig auch die Anzahl der jeweils anderen Art reduziert, sowie zusätzlich oder alternativ die insgesamt gewachsene Anzahl der T-Helfer-Zellen vermehren.

Durch einen innerhalb der Gruppe der T-Helfer-Zellen zusätzlich wachsenden Anteil eines ganz bestimmten Typs wächst deren Anzahl gegenüber dem Normalzustand einmal durch die absolute Erhöhung sowie zum anderen durch die Erhöhung des relativen Anteils an der Gesamtzahl aller T-Helfer-Zellen stark an. Dadurch wird sehr schnell eine sehr viel größere Anzahl von Plasmazellen zur Abwehrmolekülen gebildet. Also wird die Anzahl dieser Abwehrmoleküle mit insgesamt drei Faktoren verstärkt, nämlich der absoluten und der relativen Erhöhung der Anzahl von T-Helfer-Zellen sowie deren Wirkungsverstärkung durch Multiplikation in den Plasmazellen.

Ein weiterer, vorteilhafter Verstärkungsfaktor bei der Wirkung ist, dass durch die Auswahl einer der hier vorgestellten Arten vorrangig solch Plasmazellen gebildet werden, die genau die jeweils benötigten Antikörper bilden. Durch die größere Anzahl der hier präsentierten Bakterienstämme ist es möglich, denjenigen Bakterienstamm auszuwählen, der für eine bestimmte Art von Eindringling besonders wirksame Antikörper bildet. Wenn zum Beispiel ein Patient an einer Allergie leidet, ist es sinnvoll eine der Arten K1-Lb1, K1-Lb6, K2Lb4, K4-Lb6, K6Lb4 oder K9-Lb3 auszuwählen.

Zusätzlich wird das Immunsystem auf einen erneuten Kontakt mit dem Eindringling vorbereitet, indem in einem Teil der B-Zellen die charakteristischen Merkmale des eindringenden Fremdstoffes gespeichert sind, sodass beim nächsten Eindringen direkt mit der Produktion der passenden Antikörper begonnen werden kann. Diese "sekundäre Immunantwort" liegt auch den meisten Schutzimpfungen zugrunde.

Ein weiterer Vorteil der großen Anzahl von den hier vorgestellten Bakterienarten ist ihre Mehrfachwirkung bei multimorbiden Patienten, also bei Patienten, die an mehr als einer einzigen Krankheit leiden. Wenn zum Beispiel der vorerwähnte Allergiepatient zusätzlich auch noch unter einer Überfunktion der Galle leidet, so ist die Bakterienart K4-Lb6 deshalb von besonderem Vorteil, da sie eine besonders hohe Gallensalzresistenz aufweist und daher trotz dieses zweiten Problems noch die erste, erwünschte antiallergene Wirkung hat.

Eine andere, vorteilhafte Eigenschaft der hier vorgestellten Bakterienstämme ist ihre probiotische Eigenschaft. Diese Eigenschaft wird u.a. dadurch nachgewiesen, dass sämtliche Stämme aus einem seit Generationen erfolgreich genutzten Nahrungsmittel isoliert wurden nämlich aus " Kimere", einem aus Perlhirse (Pennisetum Claucum) durch Spontanfermentation hergestelltem Teig, dessen Ursprungsort der District Mbeere in Kenia ist. Da sämtliche Bakterienarten in größerer oder kleinerer Konzentration in diesem sehr weit verbreiteten und vorrangig genutzten Grundnahrungsmittel enthalten sind, ist ihre Verträglichkeit nachgewiesen. Ebenso ist damit nachgewiesen, dass sie im Vergleich zu einer sehr großen Anzahl von allopathischen Arzneimitteln als nahezu nebenwirkungsfrei einzustufen sind.

Die Auswahl von Bazillen der Art Lactobacillus fermentum wurde durch die Untersuchung der typischen Bedingungen zur traditionellen Herstellung von "Kimere" induziert, nämlich die hygienischen Umstände während der Gewinnung des Ausgangsmaterials Perlhirse, ihre Verarbeitung durch Wässern in sehr wenigen, nach unseren Maßstäben nur unvollkommen gereinigten Gefäßen, ihre Verarbeitung durch Mahlen in feuchtem Zustand mit zumeist immer wieder ein und derselben Mühle und ihre Fermentation in der Regel in ein und demselben, dafür reservierten Gefäß. Das würde nach europäischen Maßstäben eine Kontaminierung durch für den Menschen nachteilige Mikroorganismen befürchten lassen.

Die Untersuchung hat jedoch gezeigt, dass dieses ganz eindeutig nicht der Fall ist, sondern wenigsten 90% der aufgefundenen Mikroorganismen der Art Lactobacillus fermentum zugehörten. Daraus ist nicht nur zu schließen, dass diese Art generell für den Menschen verträglich ist, sondern dass sie andere, für den Menschen weniger verträgliche Mikroorganismen zumindest unterdrückt oder größtenteils eliminiert.

Bei den Untersuchungen hat sich die Population mit Mikroorganismen bei Kimere in der Regel als sehr stabil erwiesen, was ein positives Merkmal für die Nutzung der dabei vorgefundenen Bakterienarten ist. Ein weiterer Vorzug ist die relativ saure Umgebung, also ein relativ niedriger pH-Wert. Da die Mikroorganismen sich in dieser sauren Umgebung als langzeitstabil erwiesen haben, werden sie auch die saure Umgebung im Magen und im Darm überleben und auf diese Weise bei oraler Verabreichung - wie z.B. als Nahrungsergänzungsmittel - überhaupt wirksam werden können.

Dabei wurden sogar Stämme gefunden, die ein derart hohes Maß an Säurebeständigkeit auszeichnet, dass sie durch den Magen und den Dünndarm hindurch sogar bis in den Dickdarm gelangen können, um dort ihre heilsame Wirkung zu entfalten.

Von den als wirkungsvoll erkannten Bakterienstämmen wirken fast alle nicht nur gegen eine einzige Art von Beschwerden sonder gegen mehrere, sodass sie unter anderem auch für eine Behandlung von mehrfach Erkrankten geeignet sind. Dabei wurden jedoch eindeutige Schwerpunkte der Wirkungsart gefunden, sodass den einzelnen Bakterienarten gezielt ein Wirkungsschwerpunkt zugeordnet werden kann.

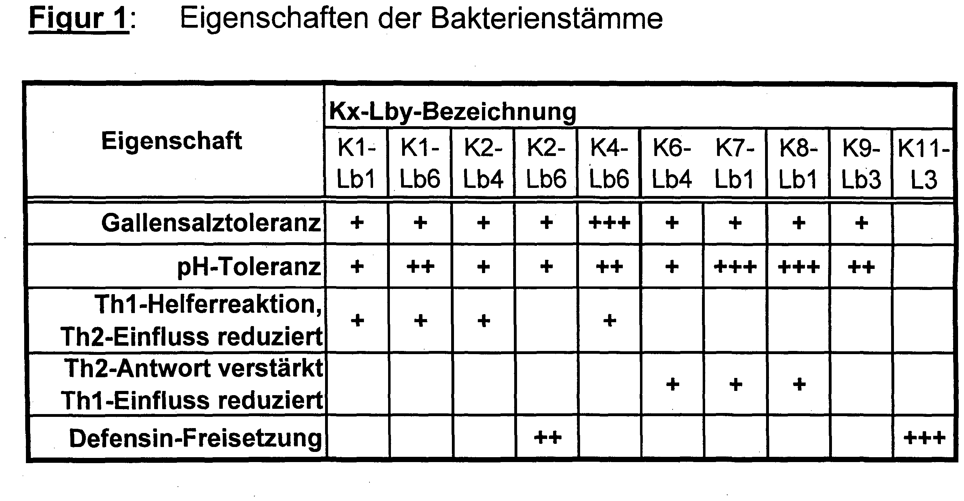

Die erste der aufgefundenen Wirkungsarten ist eine Verstärkung der Immunabwehr durch eine deutliche Verschiebung der T-Helfer-Zellen weg von den T-Helfer2-Zellen hin zu den T-Helfer1-Zellen. Mit diesem Schwerpunkt wirken vor allem die Stämme K1-Lb1, K4-Lb6. In der Figur 1 ist das Ergebnis der in vitro simulierten Wirkung niedergelegt. Daran sind auch die unterschiedlichen Intensitäten dieser Wirkungsart in jeweils drei Stufen abzulesen.

Diese Bakterienstämme sind also für Arzneimittel, Nahrungsmittelzusätze oder pharmazeutische Zubereitungen zur prophylaktischen Behandlung oder zur Reduktion des Risikos der Manifestation oder zur Therapie von T-Helfer2-getriebenen Krankheiten geeignet, wie z.B Ekzem und/oder atopischer Dermatitis und/oder Asthma und/oder Rhinitis allergica oder anderer Allergien und/oder Tuberkulose und/oder Colitis ulcerosa und/oder Eosinophiler Pneumonie.

Diese Bakterienstämme sind auch für Arzneimittel, Nahrungsmittelzusätze oder pharmazeutische Zubereitungen geeignet, die zur Prophylaxe oder Risikoreduktion oder zur Therapie bei Beschwerden, Infekten oder anderen Krankheiten einsetzbar ist, deren Abwehr Th1-vermittelt ist, wie z.B. Darminfektionen und/oder Reisediarrhöen und/oder Erkältungen und/oder Urogenitalinfekten und/oder HIV-assoziierten Komplikationen und Beschwerden und/oder Candidiasis.

Von den aufgefundenen Bakterienstämmen wirken einige mit einem gegenteiligen Akzent der T-Helfer-Zellen: sie bewirken eine Verschiebung der Antwort weg von den T-Helfer1-Zellen hin zu den T-Helfer2-Zellen. Damit werden Bakterienstämme präsentiert, die auch für ganz andere Krankheiten in der gleichen, vorteilhaften Weise nutzbar sind. Diese Wirkung zeichnet vor allem die Stämme K7-Lb1 und K8-Lb1 aus.

Diese Stämme, die vorrangig eine Verstärkung der Immunabwehr durch Verschiebung weg von den T-Helfer1 und hin zu den T-Helfer2-Antworten bewirken, sind zur Herstellung eines Arzneimittels oder eines Nahrungsmittelzusatzes oder einer pharmazeutischen Zubereitung zur prophylaktischen Behandlung oder zur Reduzierung des Risikos der Manifestation von Autoimmunerkrankungen wie Morbus Crohn oder anderen T-Helfer1-getriebenen Krankheiten geeignet.

Die vorgenannten Stämme K7-Lb1 und K8-Lb1 wirken zugleich auch hemmend gegen Entzündungen. Deshalb sind diese Bakterienstämme geeignet zur Herstellung eines Arzneimittels oder eines Nahrungsmittelzusatzes oder einer pharmazeutischen Zubereitung zur Reduktion oder Eliminierung der Auswirkungen von Autoimmunerkrankungen wie Arthritis oder Dermatiden oder von Allergien oder von Erkrankungen mit entzündlicher Komponente wie einem metabolischen Syndrom oder Atherosklerose.

Die vorgenannten Stämme K7-Lb1 und K8-Lb1 sind bei der Prophylaxe oder der Therapie von rheumatoider Arthritis, Hashimoto Thyreoiditis, Uveitis, Psoriasis, Typ1 Diabetes, Morbus Sjögren, Zoeliakie, Systemischer Lupus erythematosus, ankylosierende Spondylitis, Morbus Crohn, entzündliche Darmerkrankungen, Sclerodera, Sarkoidose, Multipler Sklerose, Vitiligo, Basedow Autoimmunthyreoiditis, endokriner Ophthalmopathie, Myasthenia gravis, Osteoarthritis, Atherosklerose und damit auch bei Herzinfarkt, anderen, peripheren arteriellen Verschlusserkrankungen, beim Hirninfarkt (Schlaganfall), metabolischem Syndrom und damit auch bei Adipositas, Hypertonie, Insulinresistenz, Diabetes Typ2, Dyslipoproteinämie sowie bei Amyotropher Lateralsklerose, interstitieller Cystitis und/oder dem Reizdarmsyndrom(IBS) sinnvoll und effizient anwendbar.

Einige der hier vorgestellten Bakterienstämme verstärken die Immunabwehr zusätzlich dadurch, dass sie eine erhöhte Freisetzung von Defensin aus den Darmzellen bewirken. Bekannt ist, dass Defensine viele kationische und hydrophobe Aminosäurereste tragen. Es sind also amphipathische Peptide. Diese positiven Ladungen interagieren mit den negativen Ladungen der Erregermembranen. Die Vorliebe der Defensine sind Membranen, die sich durch einen geringen Anteil an Cholesterol auszeichnen und sich dadurch von denen der eukaryontischen Organismen unterscheiden. Wenn sie die Membran durchdrungen haben, interagieren sie ebenfalls mit anionischen Molekülen innerhalb der Erregerzelle, etwa der DNA und RNA. Dadurch ist das Wirkungsspektrum breit und entspricht dem eines Breitbandantibiotikums, so dass es für die Erreger schwierig ist, dem Mechanismus der Defensine entgegen zu wirken.

Insoweit ähnelt der Wirkungsablauf dem der T-Helfer-Zellen, weshalb sie der gleichen erfinderischen Idee zuzuordnen sind. Diese Wirkungsart trifft vor allem für die Stämme K2-Lb6 und K11-Lb3 zu.

Eine weitere, herausragende Eigenschaft ist die Tolerierung einer 3%igen Gallensalzlösung. Dieses Merkmal trifft vor allem für den Stamm K4-Lb6 zu. Es sichert sein Überleben durch den größten Teil des menschlichen Verdauungsapparates hindurch bis in den Dickdarm. Weil das Bakterium dort noch in lebendem Zustand eintrifft, können es dort auch noch seine heilsame Wirkung entfalten, nämlich die Verstärkung der T-Helfer1-Reaktion und die Abschwächung des T-Helfer2-Einflusses.

Für eine sehr viel größere Anzahl der hier präsentierten Bakterienstämme ist zwar ein Überleben in den Gedärmen nicht mehr möglich, jedoch zumindest innerhalb des Magens. Wenigstens 8 % von ihnen überleben einen pH-Wert von 3,0 für wenigstens drei Stunden. Dadurch können sie ihre Wirksamkeit auch nach dem Durchdringen des Pförtners innerhalb des Magens noch voll entfalten. Dieses trifft für die Stämme K1-Lb1, K2-Lb6, K4-Lb6, K7-Lb1, K8-Lb1 zu.

Diese Stämme sind deshalb unter anderem bei den folgenden Beschwerden und Krankheiten sinnvoll zur Prophylaxe, zur Vermeidung eines Übergehens in einen chronischen Zustand und zur symptombezogenen Therapie von Osteoporose durch Ansäuerung des Milieus und Verdrängung anderer Keime mit der Folge der besseren Mineralstoff- und Spurenelementresorption einsetzbar. Ebenso sind sie sinnvoll bei Leberversagen und hepatischer Encephalopathie durch die Verminderung oder Hemmung der Resorption von Toxinen. vorrangig entweder auf die T-Helfer-Zellen 1 oder auf die T-Helfer-Zellen 2 lenkt.

Als Lösung präsentiert die Erfindung eine pharmazeutische und/oder dietätische Zusammensetzung, in der zur Verstärkung der adaptiven Immunabwehr mittels T-Helfer1- und T-Helfer2-Zellen Bakterien der Art Lactobacillus fermentum von wenigstens einem der Stämme K1-Lb1 oder K1-Lb6 oder K2-Lb4 oder K6-Lb4 oder K7-Lb1 oder K8-Lb1 oder K9-Lb3 und/oder der Art Lactobacillus plantarum vom Stamm K4-Lb6 und/oder zur Verstärkung der nativen Immunabwehr Bakterien der Art Lactobacillus fermentum von wenigstens einem der Stämme K2-Lb6 oder K11-Lb3 enthalten sind.

Die hier benutzten Bezeichnungen KxLBy sind aus der Systematik der ursprünglichen Untersuchung von Kimere entstanden. K steht für Kimere, der Quelle der hier vorgestellten bisher unbekannten Stämme, Lb steht für Lactobacillus. Aus der großen Anzahl von untersuchten Spezies wurden einige ausgewählt, so dass der erste Parameter der Bezeichnung- Kx - und der zweite Parameter der Bezeichnung - Lby - keine durchlaufenden Zahlen enthält.

Die Bezeichnungen KxLBy der hier präsentierten Stämme der Art Lactobacillus fermentum führen auf zwei alternativen Arten zu einer eindeutigen Identifikation des jeweiligen Bakterienstammes:
Die erste Alternative ist die bekannte Methode der Hinterlegung eines Abkömmlings der jeweiligen Art bei der DSMZ, der Deutschen Sammlung von Mikroorganismen und Zellkulturen GmbH in der Inhoffenstraße 7B in D-38124 Braunschweig. Nach der für eine Patentanmeldung vorgeschriebenen Prozedur wurden unversehrte und lebende Exemplare der hier genannten Bakterienstämme bei der DSMZ hinterlegt und zwar am 6.August 2009 unter den folgenden Zeichen:
   Lactobacillus fermentum K1-Lb1
   Lactobacillus fermentum K2-Lb6
   Lactobacillus fermentum K7-Lb1
   Lactobacillus fermentum K8-Lb1
   Lactobacillus fermentum K11-Lb3 und
   Lactobacillus plantarum K4-Lb6

Die übrigen Stämme wurden bislang noch nicht bei der DSMZ hinterlegt. Zu ihrer Offenbarung dient die zweite, alternative Methode der Bekanntgabe des PFGE-Bandenmusters.

Eine Alternativ zu der Hinterlegung von lebenden Bakterien ist die eindeutige Identifikation mittels Pulsfeldgel-elektrophoresemuster (PFGE-Muster). Für jeden der hier genannten Bakterienstämme sind in der beiliegenden Figur 3 die jeweiligen sogenannten Bandenmuster hinterlegt. Bakterien, die nach der PFGE-Methode analysiert werden und dabei ein gleiches Bandenmuster zeigen, sind damit eindeutig als dieser Stamm der jeweiligen Art identifiziert.

Eine andere Untervariante ist, dass bei wenigstens einem der Stämme die Acyltransferasen durch die Diacylglycerol-Synthese (DAG) katalysierbar ist.

In einer weiteren Variante des Wirkens der hier vorgestellten Bakterienstämme katalysieren bei wenigstens einem der Stämme Enzyme weitere Zuckertransfers zur Synthese von mikrobiellen Teptoglykanen.

Dabei kann bei wenigstens einem der Stämme durch eine Lipoteichonsäuresynthese und/oder durch eine Peptidoglycansynthese als Transporter die Ausschleusung aus dem Cytoplasma vermittelt werden.

Zu Anfang der Beschreibung wurde erwähnt, dass die hier vorgestellten Bakterienstämme durch die Hinterlegung wenigstens eines lebenden Exemplars bei der DSMZ identifiziert werden. Alternativ dient das jeweilige Bandenmuster der Pulsfeld-Gelelektrophorese als eindeutig zu identifizierendes Merkmal. Für dieses Verfahren ist es ein sinnvoller Zwischenschritt, zu überprüfen, ob die Wirkungsvermittelnden Gene ein stammspezifisches Muster der Suppressiven Subtraktiven Hybridisation (SSH) aufweisen.

Methoden und Besonderheiten der zugrunde liegenden Untersuchung:

### 1. Herkunft aus Kimere

Kimere ist eine spontanfermentierter Teig oder Brei aus Perlhirse, der nach traditionellem Verfahren erst durch trockenes und dann durch nasses Mahlen auf einem Mahlstein und anschließendes Fermentieren u.a. im Distrikt Mbeere in Kenia, Ostafrika hergestellt und verzehrt wird.

Eine Besonderheit ist, dass Kimere 18-24 Stunden vor dem Verzehr fermentiert und in seinem aktiv fermentierenden Zustand als Nahrung aufgenommen wird oder bis zu drei Tagen aufbewahrt werden. Ansonsten ähnelt es der Zubereitung des üblichen ostafrikanischen Breis, der Uji genannt wird. Kimere unterscheidet sich von Uji nur in seiner Viskosität und in seiner Konsistenz, denn Kimere ist dickflüssiger.

Die Unterschiede ergeben sich aus der Herstellungsmethode, die die mikrobielle Bevölkerung des Endproduktes beeinflusst. Die Produktion von Uji beinhaltet das mechanische Mahlen von Getreide, wie Mais, Sorgum, Hirse oder anderem mit einer Hammermühle, gefolgt von dem Vermischen des entstandenen Schrotes mit Wasser und einer spontanen Fermentation oder einem Zurückschütten eines Teils einer bereits fertig zubereiteten Uji-Portion. Zum Schluss wird das fermentierte Produkt gekocht und vor dem Verzehr gesüßt.

Im Unterschied dazu wird Kimere auf einem Mahlstein in trockenem Zustand gemahlen, woran sich meist drei Nassmahlvorgänge mit Dekantierungen und schlussendlich eine Fermentation anschließt. In einer Variante wird ein Teil des Produktes zu einem dünnen Schleim umgewandelt, indem Wasser hinzugefügt wird, und dann gekocht. Das gekochte Kimere wird dann mit dem ungekochten wieder vermischt.

Der größte Unterschied zu Uji ist, dass Kimere in seinem aktiv fermentierenden Stadium verzehrt wird und deshalb lebende Mikroorganismen enthält, wohingegen Uji sofort nach dem Kochen verspeist wird und daher keine lebenden, fermentierenden Mikroorganismen enthält. Diese Herstellungsweise von Kimere ist typisch für die Regionen Mbeere, Tharaka, Chuk und Embu östlich vom Mount Kenia.

Nach dem ersten Reinigungsvorgang und dem trockenen Schroten nimmt das gebrochene Korn leicht Wasser auf - etwa 30 % Volumenanteil - und wird bei Raumtemperatur etwa 30 bis 60 Minuten eingeweicht. Dadurch werden die gebrochenen Körner aufgeweicht und für die folgenden Nassmahlvorgänge vorbereitet.

Es folgen insgesamt drei Nassmahlvorgänge mit einem dicken Brei als Ergebnis. Zwischen jedem Nassmahlvorgang wird weiteres Wasser hinzugegeben, und nach jedem Mahlvorgang eine Dekantierung durchgeführt. Das Ergebnis ist eine milchige Suspension, von der zwei Drittel unter Umrühren gekocht werden.

Nach dem Kochen werden sie zusammen mit dem weiteren, nicht gekochten Drittel in ein Fermentationsbehältnis gegeben und für 18 bis 24 Stunden der Fermentierung überlassen. Die ungekochte Portion enthält die Mikroben, die in den Körnern enthalten waren oder die während der Mahlvorgänge aufgenommen worden sind.

Zumeist wird noch ein Teil einer vorherigen Zubereitung von Kimere hinzugegeben, was die Fermentierung beschleunigt und die letztendliche mikrobielle Bevölkerung beeinflusst. Während der Fermentierung wird durch die Lactobacillen der Anteil von Phythaten reduziert, Krankheitserreger eliminiert und die Schmackhaftigkeit verbessert.

Der Nährwert von Kimere wurde also gegenüber nicht fermentierten Produkten verbessert und ist frei von Colibakterien und anderen Enterobakterien, was ein Anzeichen von mikrobieller Sicherheit ist. Die Gegenwart von lebenden Bakterien in Kimere ist von besonderem Interesse, da es die Basis einer probiotischen Anwendung ist.

### 2. Aufnahme und Vorbereitung der Proben von Kimere

Die Proben wurden in 11 Höfen in Kathera aufgenommen, einem Teil von Kiang'Ombe, dem Evurore -Teil des Distrikts Mbeere. Die Proben wurden zubereitet und über Nacht in die Fermentationsgefäße eingelegt. Am nächsten Morgen wurden sie gesammelt und in einem Schraubglas zum Max-Rubener-Institut in Kiel gebracht, um dort bei 4° Celsius für maximal 24 Stunden gelagert zu werden. Der Transport dauerte weniger als acht Stunden, während derer die Proben bei normaler Raumtemperatur befördert wurden.

Dadurch wurde sicher gestellt, dass sich die isolierten Mikroorganismen ähnlich wie die Mikrobenbevölkerung während der sonst üblichen Fermentation verhielten, da Kimere für 18 bis 24 Stunden vor dem Verzehr fermentiert wird und noch drei Tage danach aus dem selben Fermentationsgefäß konsumiert werden kann.

Die Proben wurden sehr gut in einem Vortex-Mischer vermischt. 1 g der Probe wurde in 9 ml sterile Ringerlösung gegeben, um eine Verdünnung von 10⁻¹ zu erhalten. Dann wurden Serien von Verdünnungen gemacht bis zu 10⁻⁸ für die Zählung der Laktobacillen.

### 3. Bestimmung des pH-Wertes von Kimere

Für die Bestimmung des pH-Wertes der Kimereproben wurde die Methode der AOAC (Association of Analytical Communities) nach dem Stand von 1995 angewandt. 10g von den Proben wurden jeweils mit 40 ml doppelt destilliertem Wasser vermischt, die Mischung wurde nach einer Wartezeit von 10 Minuten mit einem Delta 320 pH-Messgerät gemessen. Die pH-Werte wurden dreifach bestimmt und der Mittelwert berechnet.

Die aerobischen Mesophilen wurden auf einem Tellerzählagar (PCA) gezählt und bei 30°C für 48 Stunden aerobisch inkubiert. Die Anzahl der Lactobacillen wurde nach dem Flächenzählverfahren auf einer MRS-agar (Merck, Darmstadt, Deutschland) gemäß dem Verfahren von De Man und anderen von 1960 bestimmt, sowie auf dem M17 agar (Merck, Darmstadt, Deutschland).

Die MRS Agarplatten wurden in einer anaerobischen Kammer (MACS MG500+TG Airlock, dw-scientific, Shipley, West Yorkshire, England) bei 37° C für 48 Stunden inkubiert. Dabei bestand die Gasatmosphäre aus 10 % Wasserstoff, 10 % Kohlendioxyd und 80 % Stickstoff.

Dann wurden die M17-Agarplatten aerobisch bei 30° C für 48 Stunden inkubiert. Nur Platten mit koloniebildenden Einheiten (Colony forming units = cfu) zwischen 10 und 300 pro Gramm wurden analysiert und das Ergebnis als dekadischer Logarithmus der cfu-Anzahl pro Grammes des Nassgewichtes der Probe aufgezeichnet.

### 4. Isolierung und biochemische Charakterisierung der Lactobacillen

1 g der Probe von Kimere wurde in 9 ml Ringerslösung abgewogen und mehrfache, zehnfache Verdünnungen wurden hergestellt bis zu 10 ⁻⁸. Von jeder Verdünnung von 10⁻⁵ bis 10⁻⁸ wurden jeweils 0,1ml zweifach auf einer MRS-Agar-Platte ausgestrichen. Die Platten wurden bei 37° C für 48 Stunden in einer aerobischen Kammer wie oben beschrieben inkubiert.

Einzelne, unterschiedliche Kolonien wurden, basierend auf ihrer Morphologie ausgewählt und durch erneutes Aufstreichen auf MRS-Agar purifiziert und mit dem Mikroskop inspiziert. Die Gram-Färbung und die Kathalasereaktion wurden so durchgeführt, wie von Harrigan und McCance 1990 beschrieben wurde.

Alle stabförmigen Gram-positiven und Kathalase-negativen Isolate wurden bei -80°C in der Cryo-Bank® vials aus Deutschland für weitere Charakterisierungen aufbewahrt. Die Produktion von CO₂ aus Glucose in MRS-Lösung wurde mit Durhamröhren bestimmt. Die chemische Charakterisierung der Ketten wurde mit API 50 CH Kits mit API 50 CHL Medium in Übereinstimmung mit den Richtlinien des Herstellers Biomerieux, Nürtingen, Deutschland und durch die Fermentation von verschiedenen Carbohydraten in MRS-Lösung durchgeführt. Die Isolate wurden auch auf ihre Fähigkeit zu Wachstum bei 15°C und 45°C geprüft.

### 5. Molekulare Charakterisierung der Laktobacillen mit der verstärkten ribosomalen DNA Restriktionsanalyse (ARDRA)

Das DNA-Material für die Polymerase-Kettenreaktion (Polymerase Chain Reaction = PCR) wurde nach der Methode von Ismail 2007 und Vaneechoutte et al. 1992 vorbereitet: Eine Kolonie von einer MRS-Agarplatte wurde in 500 µl PCR-Pufferlösung aufgelöst, um eine trübe Suspension herzustellen (Mc Farland 3) und in einem Thermomixer bei 95°C für zehn Minuten inkubiert. Dieses DNA-Material wurde bei -20°C gelagert bis die PCA-Analyse ausgeführt wurde.

Die PCR wurde in einem Eppendorf Mastercycler 5330, Hamburg, Deutschland, in Volumeneinheiten von 50 µl ausgeführt, bestehend aus 20 µl PCR Mastermischung (Fermentas, Sankt Leon-Roth, Deutschland), 26 µl doppelt destilliertes Wasser, 1 µl des jeweiligen Primers und 2 µl der DNA-Probe. Das PCR-Programm war: Initiale Denaturierung bei 93°C für fünf Minuten, 35 Zyklen von Denaturierung bei 92°C für eine Minute, Aushärten bei 58°C für 1,5 Minuten und Verlängerung bei 72°C für 2,5 Minuten respektive, gefolgt von einer finalen Verlängerung bei 72°C für zehn Minuten und Kühlung und Halten bei 4°C.

Diese Sequenzen von oligonukleotiden Primern waren: Forward-UP68 5'-TGG CTC AGA TTG AAC GCT GGC GGC-3' und Reverse -UP69 5'-CCT TTC CCT CAC GGT ACT GGT-3'. Eine Verstärkung von etwa 2.4 Kb wurde erzeugt, die größtenteils aus der 16S rDNA, der 16S-23SrDNA Abstandsregion und Teilen von der 23SrDNA bestand (Ismail, 2007, Vaneechoutte et al 1992). Die Restriktion wurde über Nacht bei geeigneten Temperaturen in 20 µl Volumeneinheiten ausgeführt, die 0,5 µl Ristriktionsenzym enthielten (HaeIII.,HinfI.) (Fermentas) Ddel (New England Bio Labs Inc.), 2µl Enzympufferlösung (Buffers R für HaeIII. und Hinfl und Buffer 3 für Ddel), 2,5 µl-15,5 µl 1xTE Puffer, und 2,5 -15 µl PCR-Produkt.

Das genutzte Volumen basierte auf der Stärke des Signals von den PCR-Produkten, die auf dem Muster des Agarose-Gel-Restriktions fragment analysiert wurden nach der Elektrophorese in 1,5 %-iger Agarose-Gel in 1xTAE Puffer bei 80 V für zwei Stunden. Die Färbung wurde mit Ethidiumbromid für 30 Minuten durchgeführt, gefolgt von einer Waschung während 15 Minuten in Wasser. Die Fotografien wurden unter UV-Licht aufgenommen.

### 6.Stammspezifische PCR für Lactobacillum fermentum

Die stammspezifische PCR wurde nach der Methode ausgeführt, die Dickson und andere 2005 beschrieben haben. Dabei wurde primäres LF1 (nt196-215; 5'-AAT ACC GCA TTA CAA CTT TG-3') und LF2 (nt529-510;5'-GGT TAA ATA CCG TCA ACG TA-3') spezifisch für Lactobacillum fermentum benutzt. Eine Verstärkung von 337bp Länge wurde erzeugt. Das Verstärkungsprogramm wurde ohne Änderung angewandt. Die PCR-Produkte wurden so analysiert, wie es für ARDRA beschrieben worden ist.

### 7.16S rDNA Sequenzen:

Basierend auf den jeweiligen ARDRA Profildaten (Tafel 2-2) wurden 12 Stämme für das Sequenzieren ausgewählt. Die Stämme wurden auf der Basis der Profile ausgewählt, die nach der Anwendung des Hinfl-Ristriktions-Enzyms erhalten worden waren. Fünf Stämme, die jedes der beiden ARDRA Profile (A1 und A2) repräsentierten und jeweils eines der Profile B und C. Die PCR Produkte, erzeugt mit den Primern UP68 (Vorwärts) und UP 69 (Rückwärts) (Vaneechoutte et al 1992), wurden mit einem Clean-Up-Kit (NucleoSpin® Extrakt II, Macherey-Nagel, Düren, Deutschland) gemäß der Betriebsanleitung des Herstellers purifiziert. Die Purifizierung wurde durch Elektrophorese in 1 %igem Agarosegel kontrolliert. Eine partielle Sequenzierung von 16S rDNA wurde mit Nutzung von UP68 als Sequenzierungsprimer ausgeführt. Die Sequenzierung wurde bei MWG Biotech AG in Ebersberg in Deutschland durchgeführt.

### 8. Pulsfeld-Gelelektrophorese (PFGE)

Die PFGE-Analyse von Lactobacillus-fermentum-Stämme wurde nach dem Verfahren von Hoppe-Seyler et al. 2003 ausgeführt. Die Ristriktionsenzyme, die getestet wurden, waren NotI, AscI und SmaI. AscI ergab die besten Profile und wurde in dieser Studie benutzt. Die Elektrophorese wurde in einem Bio-Rad CHEF-DRII System ausgeführt, das auf die folgenden Bedingungen eingestellt wurde: Temperatur 14°C, Pumpengeschwindigkeit 70 min⁻¹, erste Schaltzeit 2,0 Sekunden, finale Schaltzeit 30 Sekunden, Laufzeit 24 Stunden und Spannung 175 Volt. Nach der Elektrophorese wurde das Gel in Ethidiumbromid (0,5 mg/l) für 30 Minuten eingeweicht. Das Gel wurde dann in destilliertem Wasser für 10 Minuten gewaschen und unter UV-Licht fotografiert.

### 9. Datenanalyse

Die mikrobiologische Qualität der koloniebildenden Einheiten (colonie forming units = cfu) und der pH-Wert wurden unter Gebrauch der SAS Version 9.1 analysiert. Die ARDRA Gele wurden visuell analysiert und die Muster mit denen typischer Stämme verglichen und zur Übereinstimmung gebracht. Die Ausrichtung der 16S rDNA-Sequenzen und die Erstellung des Dendrogramms wurden auf der Basis von Escherichia coli ATCC11775T (Zugangsnummer X80725) als Wurzel des Baumes (Tamura et al.2007) mit der MEGA4 Nachbarverbindungsmethode ausgeführt.

Die Sequenzen wurden auch mit den Sequenzen in der Genbank für die DNA-Daten unter Benutzung der BLAST-Algorhythmen (Altschul et al., 1997) verglichen. Die PGGE-Profile wurden unter Gebrauch des GelCompar-II-Programmes (Applied Maths, Kortrijk, Belgien) analysiert. Gleichheiten zwischen verschiedenen Ketten wurden unter Benutzung des Dice-Koeffizienten abgeleitet. Die nicht gewichtete Paar-Gruppen-Methode mit Durchschnittsverbindungen (UPGMA) wurde benutzt, um verschiedene Profile zu clustern.

### 10. Mikrobiologische Qualität von Kimere

Lactobacilli machen den größten Teil der mikrobielle Bevölkerung von fermentiertem Kimere aus. Colibakterien und andere Enterobakterien wurden weder in 0,1 Gramm von der Probe bei der Prüfung in LST nachgewiesen, noch in 0,01 Gramm der Probe auf VRB agar, und auch nicht in 0, 01 Gramm von der Probe auf VRBD.

### 11. Biochemische Charakterisierung der Lactobacillen

48 Stämme von isolierten Lactobacillen wurden auf Wachstum bei 15°C und bei 45°C und auf ihre Gasproduktion aus Glucose sowie auf die Fähigkeit, verschiedene Zucker zu metabolisieren, überprüft. Zusätzlich wurden sie durch API 50CHL charakterisiert. Alle Stämme waren in der Lage auf Fructose, Galactose, Glucose, Laktose, Maltose, Mannose, Ribose und Sucrose zu wachsen. Alle Stämme mit Ausnahme von K1Lb5 waren in der Lage auf Melibiose und Raffinose zu wachsen. Alle Stämme mit Ausnahme des isolierten K4-Lb6 waren nicht in der Lage auf Mannitol, Melecitose, Rhamnose, Sorbitol und Trehalose zu wachsen. Nur drei Isolate waren in der Lage auf Cellobiose zu wachsen. Die meisten Stämme waren in der Lage bei 45°C zu wachsen mit Ausnahme von K4-Lb6.

Durch die Identifizierung nach API 50CHL konnten 33 Stämme von Lactobacillus fermentum isoliert werden. Die API-Identitätspunkte reichten von 38, 9 bis 99, 9 %.

### 12. PFGE-Profile der Kimere-Isolate

Alle Isolate aus Kimere, die als Lactobacillus fermentum identifiziert wurden, wurden einer PFGE-Analyse unterzogen. Von den Restriktionsenzymen, mit denen getestet wurde (AscI, NotI, SmaI) ergab nur AscI zum Vergleich geeignete Ergebnisse. Figur 3 zeigt die Profile der hier offenbarten 10 Stämme. Obwohl sie von einer einzigen Art von fermentierter Nahrung abisoliert worden sind, zeigen die meisten Arten ziemlich verschiedene PFGE-Muster, was die hohe Biodiversifizierung innerhalb der Stämme des Lactobacillus fermentum aus Kimere verdeutlicht.

Die recht deutlichen Unterschiede der Bandemuster zwischen den einzelnen Stämmen plausibilisieren auch, dass mit diesem Bandemuster eine eindeutige Identifikation möglich ist.

### 12.Stammspezifische Immunmodulation der aus Kimere isolierten Stämme von Lactobacillus fermentum

Es zeigt sich, dass probiotische Effekte stammspezifisch sind. Bisher haben jedoch sehr wenige Studien Stämme derselben Art miteinander verglichen, um zu diesem Schluss zu kommen. Mit der hier vorliegenden Studie sollte das probiotische Potenzial von Stämmen der Art Lactobacillus fermentum untersucht werden und stammspezifisch in vitro demonstriert werden. Neun Stämme von Lactobacillus fermentum und ein Stamm von Lactobacillus plantarum, die in einer 3%igen Gallensalzlösung wachsen konnten, wurden aus 48 Stämmen nach einem Gallentoleranztest ausgewählt.

Diese Stämme wurden weiter untersucht, nämlich auf ihre Wachstumsgeschwindigkeit in einem MRS Medium (Man Rogosa und Sharpe) ergänzt mit 0,3iger und 3%iger Gallensalzlösung und auf ihren Widerstand gegen einen niedrigen pH-Wert durch das Zählen der überlebenden, kolonieformenden Einheiten (cfu) nach der Exposition in pH2 und pH3 für drei Stunden.

Das immunmodulierende Potential wurde durch Mitinkubation der Stämme mit menschlichen, peripheren Bluteinzelkernzellen (PBMCs) für 48 Stunden bei 37°C und Messung der Zytokine untersucht und zwar auf Th1-Cytokine (Interferon-γ) und Th2-Zytokine (Interleukin-4) in den Überständen durch enzymatisch verbundene, immunsorbierende Prüfung (enzymatic-linked immunosorbent assay = ELISA).

Alle Stämme konnten 0,3%ige Gallensäure ertragen, wobei der Stamm K4-Lb6 sogar 3,0%ige Gallensäure aushalten konnte. Die Stämme K7-Lb1 und K8-Lb1 zeigten sogar eine Überlebensrate von rund 53 % bzw. 27, 2 % bei einem pH-Wert von 2,0; wohingegen die meisten Stämme nur bei einem pH-Wert von 3,0 nicht versehrt wurden.

Wie bei LGG reduzierten die Stämme K7-Lb1 und K8-Lb1 Interleukin-4. LGG und alle anderen Stämme induzierten einen Wechsel von dem basalen Th1/Th2-Verhältnis in Richtung von Th1 mit Ausnahme der Stämme K7-Lb1 und K8Lb1, die einen Wechsel zu Th2 induzierten. Das SEA-Stimulierte Th1/Th2-Verhältnis wurde von den Lactobacillus-fermentum-Stämmen reduziert, wohingegen es von LGG angehoben wurde.

Die Studie zeigt also stammspezifische Besonderheiten der Stämme von Lactobacillus fementum und zeigt auch die Fähigkeit, die von Staphylococcus enterotoxin A (SEA) induzierte Produktion von Interferron γ zu unterdrücken, eine Eigenschaft, die von früheren Studien über Lactobacillus fermentum und von den meisten anderen Lactobacillen nicht berichtet worden ist. Diese verschiedenen Eigenschaften können genutzt werden um Krankheiten zu bekämpfen, die von einer Th1-Antwort bzw. einer Th2- Antwort getrieben werden.

### 13. Fermentierte, probiotische Nahrungsmittelzusätze und ihre Wirkung auf das Immunsystem

Die Anwendung von Milchsäurebakterien (Lactic Acid Bacteria = LAB) für die menschliche Ernährung ist seit Jahrtausenden bekannt - wo immer fermentierte Milch oder andere fermentierte Nahrungsmittel genossen wurden. Ihre direkte Verbindung zur menschlichen Gesundheit wurde erstmals 1908 von dem russischen Nobelpreisträger Elie Metchnikoff beobachtet, der die Gesundheit und Langlebigkeit bulgarischer Hirten den Kleinlebewesen in dem häufig von Ihnen verzehrten Joghurt zuschrieb.

Die Beobachtungen fanden erneutes Interesse, als die Anwendung von Lactobacillen und Bifidobakterien als probiotische Nahrungsmittelzusätze eine Bedeutung erlangten. "Probiotisch" ist definiert als "eine Nahrungsergänzung mit lebenden Mikroben, die beim Aufnehmenden das Gleichgewicht seiner in ihm lebenden Mikroorganismen vorteilhaft beeinflusst. (Fuller, R,1989, Probiotics in Man and Animals. Journal of Applied Bacteriology 66, 365-378).

Die Definition und Anwendung von "Probiotics" entwickelte sich weiter, sobald eine wissenschaftliche Evidenz gewonnen worden war (Schrezenmeir, J. und de Vrese, M., 2001, Probiotics, Prebiotics und Synbiotics, approaching a definition. American Journal of Clinical Nutrition 73,361-364.) Ein neuerlicher Vorschlag von Galdiano, C. M., de LeBlanc, A.D., Vinderola, G., Bonet, A.E.B. und Perdigon, G.,2007, Proposed Model: Mechanisms of immunomodulation induced by probiotic bacteria. Clinical and Vaccine Immunology 14, 485-492) schließt Immunmodulation in die Definition von Probiotics ein. Sie definierten Probiotic als "lebende Mikroorganismen, die bei der Zugabe zu Nahrungsmitteln die Komposition und die Aktivität von den Mikroben in dem Verdauungssystem beeinflussen, die entzündlichen Reaktionen modulieren, die nicht spezifischen Barrieren im Darm verbessern und die Immunantwort der Schleimhäute und des Systems verstärken oder verbessern."

Die Auswahl von Stämmen zur Anwendung als Probiotics konzentriert sich auf zwei wesentliche Eigenschaften: Die Anpassungsfähigkeit des Stammes selbst und den Gesundheitsunterstützenden oder funktionellen Aspekt. Diese Auswahlschemata schließen ein: Überleben in einer Umgebung mit niedrigen pH-Wert, Wachstum in Gegenwart von Gallensalzen, Adhäsion an darminnere Epithelzellen, Kolonisierung innerhalb des Verdauungssystems, Aufrechterhaltung des mikrobischen Gleichgewichtes, Nichtpathogenität gegenüber dem Aufnehmenden, Beständigkeit gegen technologische Belastungen bei der Verarbeitung und Verteilung und schließlich die Fähigkeit zur Verbesserungen der Gesundheit des Aufnehmende. (FAO/WHO, 2002, Guidelines for the evaluation of Probiotics in food. Joint FAO/WHO Working group report on Drafting Guidelines for the evaluation of Probiotics in food ftp://ftp.fao.org/es/esn/ food/wgreport2. pdf, 1-11).

Dabei muss bemerkt werden, dass nicht jeder einzelne, probiotische Stamm alle diese Eigenschaften aufweisen muss. Nach neueren wissenschaftlichen Erkenntnissen müssen Bakterien nicht "lebendig" sein, um immunmodulierende Effekte zu bewirken, denn sowohl lebende wie auch abgestorbene Bakterien oder nur die bakterielle DNA zeigte nachweislich einigen gesundheitlichen Nutzen.

Ein Überleben im Verdauungssystem ist jedoch essentiell, um sicher zu stellen, dass die Probiotics den Zielort in aktivem Stadium erreichen, abhängig von den funktionalen Erfordernissen, wie z.B. einer Kolonisierung des Verdauungssystems und dem Ausschluss von Fäulnisbakterien oder Pathogenen.

Eine Immunmodulation durch Probiotics wird durch Interaktionen mit den Immunzellen des Aufnehmenden und die Absonderung von verschiedenen Signalmolekülen wie Cytokinen und Immunglobulinen erreicht. Die T-Helfer-Zellen spielen innerhalb der Immun-Kompetenten Zellen eine herausragende Rolle bei der Immunmodulation, die durch Probiotics Cytokine produziert.

Bei der Interaktion mit den Antigenen unterscheiden sich vorhandene T-Helfer-Zellen auf verschiedene Arten, abhängig vom Typ und der Anzahl der Antigene. Die Umgebung der Cytokine bestimmt letztendlich das Verwandtschaftsverhältnis und das Wirkungsprofil. Die Unterscheidung in T-Helfer1-Zellen hängt von der Interleukin 12 bewirkten Aktivierung von Transkription 4 (STAT4) ab, die zu der Interferon-γ-Produktion führt, welche durch eine Serie von Kaskaden zu einem weiteren Heranwachsen von T-bet geführten Transkriptionsfaktoren führt, die zu einem weiteren Anwachsen von Interferon-γ, Interleukin-12-Rezeptoren β2, Interleukin-4-Unterdrückung und der Aufrechterhaltung von Verwandtschaftsbeziehungen zu T-Helfer1-Zellen führt.

Andererseits unterscheiden sich T-Helfer2-Zellen in der Gegenwart von lnterleukin-4 durch STAT 6, ein Anwachsen der Interleukin-4-Produktion, die Unterdrückung der Interferon-γ-Produktion und also einer Aufrechterhaltung der T-Helfer2-Abstammung.

Die andere Art der Unterscheidung von T-Helfer-Zellen ist die T-Regulation (Treg), die lnterleukin-10 produziert. Treg-Cytokine IL-10 regulieren das Immunsystem sowohl durch die Unterdrückung von T-Helfer1- als auch von T-Helfer2-Zytokinen, indem sie periphere Toleranzen sicherstellen. Die vierte bekannte Art von natürlichen T-Helfer-Zellen ist die Unterscheidung durch Interleukin-23, das zur Produktion von Interleukin-17 führt.

Es wird davon ausgegangen, dass ein Ungleichgewicht zwischen den T-Helfer1-, den T-Helfer2, den Treg- und den T17-Zellen der Vorläufer zu einer Manifestation von verschiedenen krankheitsfördernden Bedingungen ist. Die probiotische Funktion hebt die Zytokin-Produktion in Richtung auf eine Verstärkung der Immunabwehr eine reduizierte, allergische oder autoimmune Reaktion und/oder eine geringere Entzündungsreaktion.

Eine Modulation der Zytokinproduktion durch Probiotics in menschlichen und tierischen Modellen wurde sowohl in vitro (Pochard, E., Gosset, P. , Grangette, C., Andre, C., Tonnel, A.B. , Pestel, J., Mercenier, A., 2002, Lactic Acid Bacteria inhibit T(H)2-cytokine-production by Mononuclear cells from allergic patients. Journal Of Allergie and Clinical Immonologie 110, 617-623.
sowie Ghadimi, D., Folster-Holst, R., de Vrese, M., Winkler, P., Heller, K.J., Schrezenmeir,J., 2008, Effects of probiotic bacteria and their genomic DNA on TH1/TH2-Zytokine production by peripheral blood mononuclear cells (pbmcs) of healthy and allergic subjects, Immunobiology 213, 677-692)
sowie in vivo (Kopp, M.V., Goldstein, M., Dietschek, A., Sofke, J., Heinzmann, A. und Urbanek, R., 2008, Lactobacillus GG has in vitro effects on enhanced Interleukin-10 and Interferon-γ-release of mononuclear cells but no in vivo effects in supplemented mothers and ther neonates). Clinical and Experimental Allergy 38, 602-610).

In den meisten Fällen wurde eine Mischung von Th1-, Treg- und Th2-Cytokinen nachgewiesen, obwohl etliche Autoren einen Wechsel von Th1 oder Th2 mit in den Wirkungsablauf einschließen. Diese Immunmodulation hängt von der Dosis und der Zeit ab und scheint auch Abhängigkeiten von der Impfung der Stämme aufzuweisen.

### 14. Bakterienkulturen

Die Bakterienstämme, die in dieser Studie genutzt wurden, sind aus Kimere, einem fermentierten Teig aus Kenia, isoliert und nach molekularen Methoden charakterisiert und bei -80° Celsius in einer MAST Cyrobank® (MAST Diagnostic, Reinfeld, Deutschland) aufbewahrt. Als positive Vergleichskontrollen für T-Helfer1-Cytokine und T-Helfer2-Cytokine wurden die Stämme Lactobacillus rhamnosus GG (ATCC 5310, ein Stamm von dem bekannt ist, dass er Interferron- γ stimuliert) und Escherichia coli TG1 (BU-00035, ein Stamm von dem bekannt ist, dass er Interleukin 4 stimuliert) genutzt. Sie wurden aus kommerziellen Quellen beschafft.

Die Lactobacillen wurden über Nacht in MRS-Lösung bei 37°C anaerobisch in einer MACS-VA500 Arbeitsstation (Don Whitley Scientific Limited, Großbritannien) gezüchtet. Die Escherichia coli TG1 wurden aerobisch über Nacht bei 37° in einer Luria-Erthani (LB) Lösung gezüchtet. Die Zellen wurden bei 14.000 g (entsprechend 144.500 min⁻¹ auf einer Eppendorf Minispin-Plus Zentrifuge) für zwei Minuten zentrifugiert. Die bakteriellen Pellets wurde zweimal gewaschen mit einer phosphatgepufferten Salzlösung (PBS) aufgelöst in 1 ml PBS, enthaltend 20% Glycerin, dann in einer Zählkammer vom Verbesserten Neubauer-Typ gezählt, und anschließend auf eine Konzentration von 10⁸ Zellen pro Milliliter in einer PBS-Lösung mit 20% Glycerin gebracht und bei -80° Celsius bis zur Verwendung aufbewahrt.

### 15. Widerstand gegen Gallensalze:

48 Isolate von Lactobacillen wurden nach der bereits beschriebenen Methode mit geringen Änderungen auf ihre Widerstandsfähigkeit gegen Gallensalze geprüft. Über Nacht wurden 0,1 ml einer MRS-Wachstumkultur zu 9,9 ml MRS-Lösung hinzugefügt mit 0,3 oder 0,5 oder 1,0 oder 2,0 oder 3%iger Rindergalle (Siegmar). Sie wurden 24 Stunden bei 37° Celsius inkubiert. Das Wachstum wurde nach einer Sichtprüfung der Trübung der Röhren verzeichnet.

Zehn Isolate wurden ausgewählt, die auch bei 3%iger Ochsengalle noch ein Wachstum zeigten. Ihr Wachstum wurde dann mit 0,3%iger und 3%iger Ochsengalle überwacht. 2 ml als Aliquot (Teüprobe) von Lactobacillen aus einer frischen, über Nacht gewachsenen Kultur wurden als Kügelchen in PBS suspendiert und 100 µl davon in eine MRS-Lösung eingeimpft, die mit 0,3%iger (w/v) Gallensalz versetzt worden war. Eine 0,3%ige (w/v) Gallensalzkonzentration ist als physiologisch relevant eingestuft worden und dient in vielen Studien für die probiotische Selektion. Ebenso eine 3%ige Ochsengalle (w/v).

Die Kulturen wurden bei 37° Celsius im Wasserbad inkubiert. Das Wachstum wurde stündlich durch die Messung der Absorbierung bei 620 nm (A Index 620 nm) beobachtet. Die Versuche wurden 3-mal wiederholt. Die Absorbtionswerte wurden über die Inkubationszeiten aufgezeichnet, wie von Gilliand und Walker beschrieben worden ist. (Gilliand, S.E. und Walker, DK 1990, Factors to Consider when selecting a Culture of Lactobacillus-Acidophilus as a dietary adjunct to produce a Hypocholesterolemic Effect in Humans, Journal Of Diary Science 73,905-911).

### 16. Säurebeständigkeit

2 ml einer über Nacht gewachsenen Kultur wurden bei 14.000 g (145.400 min⁻¹ auf einer Eppendorf Minispin-Plus Zentrifuge) für eine Minute zentrifugiert. Die Pellets wurden 2-mal gewaschen mit 2ml Ringer's Lösung und nochmals suspendiert in 1,5 ml Ringer's Lösung. 0,1 ml der Suspension wurde auf 5 ml von 0,35%iger Nacl-Lösung übertragen (ergänzt mit Pepsin (0,1 Gramm pro 50 ml) und justiert auf die verschiedenen pH-Werte 2,0 und 3,0 und 6,5 mit HCl) gefolgt von einer Inkubation bei 37° Celsius in einem Wasserbad.

Nach drei Stunden wurden 5 ml von 0,1 M Phosphatpufferlösung bei einem pH-Wert von 6,5 hinzugefügt. Die Überlebensraten wurden durch die Zählung auf MRS-Agar nachgewiesen. Die koloniebildenden Einheiten (cfu) wurden in ihrem prozentualen Anteil an den Kontrollwerten ausgedrückt, die bei der Inkubation mit einem pH-Wert von 6,5 erhalten worden sind.

### 17. Test der Immunmodulation mit PBNC's (Periphere, einkernige Blutzellen)

Von gesunden Spendern in einem Alter zwischen 21 und 52 Jahren wurde vollständiges Blut gesammelt und mit EDTA (Ethylen-Diathermin-Etetra-saurer Säure) zwecks Antikoagulation vermischt. Individuen, die von einer Allergie berichteten oder kürzlich Infektionen der Atemwege erlitten hatte oder Medikamente einnahmen, wurden ausgeschlossen. Das Anwerben der Probanden und das Ziehen der Blutproben wurde nach den strikten ethischen Gesichtspunkten durchgeführt, die von dem ethischen Komitee der Universität Kiel bei der Nutzung menschlicher Probanden in der Forschung festgesetzt worden sind. Eine schriftliche Zustimmung wurde von allen Testpersonen vor ihrer Einschreibung zu dieser Studie erhalten. Die Proben wurden bei Raumtemperatur (18-25° Celsius) bis zur Isolation der PBMC's aufbewahrt.

Die PBMC's wurden wie zuvor beschrieben isoliert. Blut wurde mit Natriumchlorid (NaCl) gemischt und bei einem Mischungsverhältnis von 1:1 in 50 ml Zentrifugeröhren abgefüllt und auf einen Ficoll-Lymphoprep Plus aufgesetzt (Axis-Shield PoC AS, Oslo, Norwegen) bei einem Verhältnis von 1:2 (16 ml Ficoll und 32 ml Blut) nach einer Zentrifugierung bei 179,5 g (1.300 min⁻¹ )auf einer Eppendorf Zentrifuge 5810 R, r gleich 9,5 cm) für 30 Minuten bei 18°C. Die PBMC-Schichten wurde in sterile Zentrifugenröhrchen mit 50 ml Inhalt abgefüllt. Waschflüssigkeit (PBS plus 10% FCS) wurde hinzugefügt, um die Röhre zu füllen (etwa 25 ml) und dann bei 179,5 g für 10 Minuten bei 4° Celsius zu zentrifugieren.

Die Waschung wurde 3-mal ausgeführt mit PBS plus 10% FCS, jedes mal wurde die Hälfte des Waschmediums abgestoßen und zum Schluss das gesamte Waschmedium entfernt. Nach der dritten Waschung wurde die PBMC's im vollständigen Medium suspendiert (RPMI 1640 Medium + 10% FCS + 1% (Penicellin + Streptomycin)), alles geliefert von Gibco, Karlsruhe, Deutschland. Die PBMC's wurde in einer Zählkammer gezählt, 10 µl : 90 µl, PBMC's : Färbemittel und ausgedrückt als PBMC's / ml des Mediums.

### 18. Koinkubation der PBMC's und der Bakterien:

Die Konzentration von PBMC's, die benutzt wurde, war 1x10⁶ Zellen/ml und der Bakterien 2x10⁷ Zellen/ml (1:20 PBMC zu Bakterienverhältnis). Neben der grundlegenden Bedingung (Medium) wurde mit dem Einsatz von Staphylococcus enterotoxin A(SEA) ein stimulierender Zustand bei einer Konzentration von 2 µg/ml gemessen. Die Behandlungen waren: PBMC's + Medium als Vergleichsgröße; PBMC's + SEA + Medium; PBMC's + Bakterien + Medium und PBMC's + SEA + Bakterien + Medium jeweils in 1ml Volumeneinheiten in einer Platte mit 24 Vertiefungen und in Verdopplungen. Nach einer Inkubation bei 37° C für 48 Stunden wurde der Überstand bei 179,5 g, 4° Celsius für zehn Minuten zentrifugiert und dann durch sterile Mikrofilter mit 0,2 µm Maschenweite gefiltert und in Eppendorf Röhren (1,5 ml) in Volumeneinheiten von 250 µl gefüllt und aufbewahrt bei -20°C bis zum ELISA-Test aufbewahrt.

### 19. Cytokinprobe

Überprüfungen zum Nachweis von Interleukin und Interferon-γ in den Überständen wurden mit einer ELISA-Platte mit 96 Vertiefungen entsprechend den Anweisungen des Herstellers durchgeführt. Alle ELISA Reagenzien wurden von Mabtech AB, Hamburg, Deutschland, gekauft. Die unteren Nachweisgrenzen waren 1 pg/ml für Interleukin und 4, 2 pg/ml für Interferon-γ, wie für jeden Kit empfohlen.

Die Absorption wurde bei 450 und 570 nm Wellenlänge in einem Mikroplattenlesegerät, Molekular Devices, München, Deutschland, gemessen. Zur Korrektur der Wellenlänge wurde der Ablesewert der optischen Dichte bei 570 nm von dem für 450 nm abgelesenen Wert abgezogen. Die mittleren Absorptionswerte der leeren Platte (Hintergrundkontrolle) wurden von den Standardwerten für die Standardprüflinge und den Werten der Proben abgezogen und dann eine Standardkurve unter Gebrauch des Kurvenerstellungsprogramms mit vier Parametern und der Ableitung der Zytokinkonzentration in den Proben erzeugt.

### 20. Statistik

Mittels ANOVA wurden die Mittelwerte der Gallenresistenz von verschiedenen Stämmen miteinander vergleichen. Für die Daten zur Immunmodulation wurde die Analyse in zwei Schritten ausgeführt: Zuerst wurden alle Stämme und die Vergleichsprüflinge miteinander verglichen. Im zweiten Schritt wurden die Werte der Kontrollgrößen LGG und TG1 ausgeschlossen, die als "Ausreißer" für Interferon-γ und Interleukin-4 eingestuft wurden.

Mit dem allgemeinen, linearen Modell der SPSS 9.0 Berechnungssoftware wurden die Mittelwerte der SEM's abgeleitet und die Mittelwerte von verschiedenen Stämmen mittels des "Mann-Whitney U-Testes" miteinander verglichen. Mit Sigma Plot 11.0 wurden die Ergebnisse graphische präsentiert. Statistische Signifikanz wurde bei P>0,05 in Betracht gezogen. Um die Richtung der Verschiebung des Verhältnisses von T-Helfer1- zu T-Helfer2-Werten bei der Immunantwort zu definieren, wurde das Verhältnis von Interferon-γ zu Interleukin-4 benutzt. Das mittlere Verhältnis wurde zu 100% definiert und die Werte für die anderen Prüflinge als prozentualer Anteil an diesem Vergleichswert ausgedrückt. Werte über 100% wurden als eine T-Helfer1-Verschiebung eingestuft und Werte kleiner als 100% als eine T-Helfer2-Verschiebung.

### 21. Widerstand gegen Gallensalze und niedrigen pH-Wert

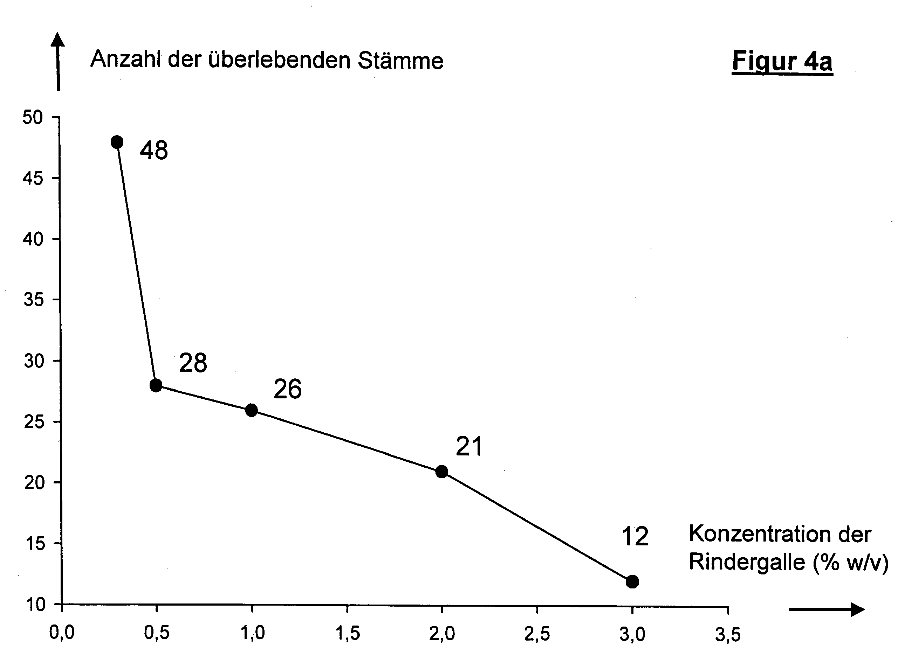

Zur Überprüfung ihre Gallentoleranz wurden alle 48 Isolate jeweils über Nacht in ein MRS-Medium eingegeben, das stufenweise auf 0,3%, 0,5%, 1%, 2% und dann auf einen Anteil von 3% Ochsengalle (w/v) gebracht wurde. Durch Überprüfung der mittleren Trübung wurde das Wachstum von zuerst 48, dann 28, 26, 21 und 12 Isolaten festgestellt(siehe Figur 4a).

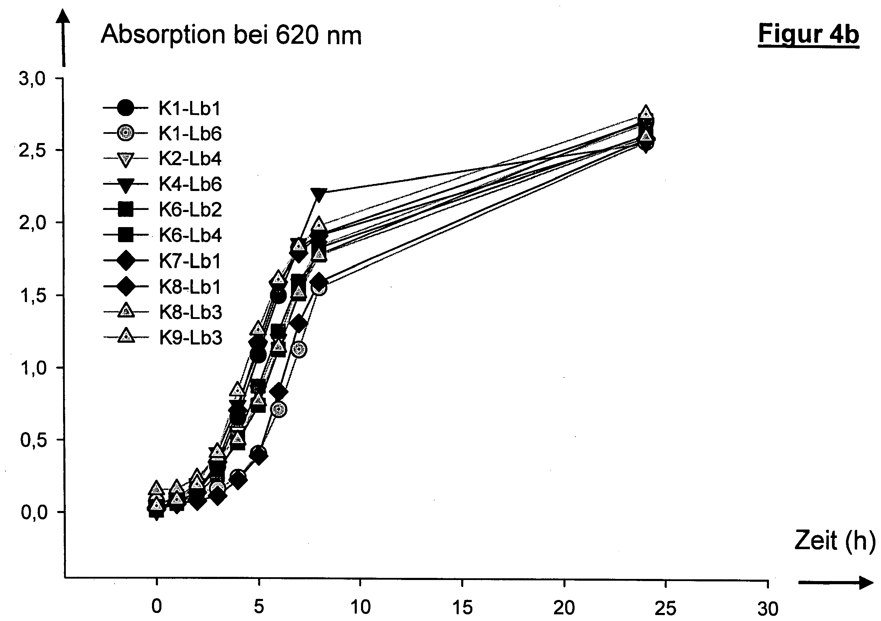

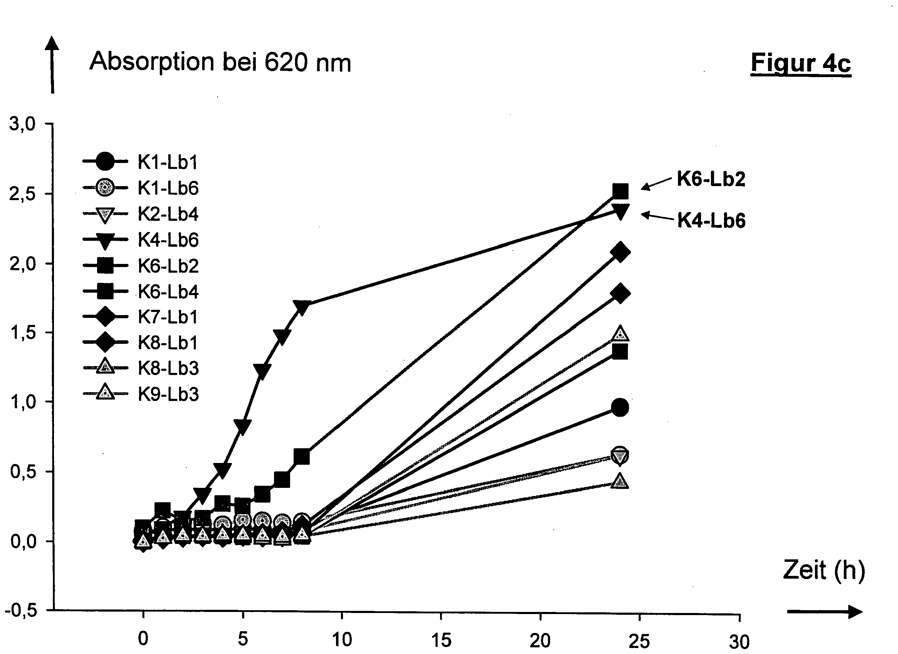

Die Überwachung ihrer Wachstumsbewegung (Wechsel in OD_{620 nm}) in MRS-Lösung, ergänzt mit 0,3%iger Ochsengalle (w/v) zeigte ein gutes Wachstum aller Stämme, was durch eine kurze Verzögerungsphase angezeigt wurde (Figur 4b). Nur die Stämme K1-Lb6 und K8-Lb1 benötigten mehr als drei Stunden um 0,3 Einheiten von A _{620 nm} zu erreichen. Wie in Figur 4c gezeigt, unterschied sich das Wachstum des Stammes K4-Lb6 signifikant von dem Mittelwert der anderen in einer Lösung mit 3%iger Ochsengalle (w/v). Der Stamm K4-Lb6 begann fast ohne Verzögerungsphase zu wachsen. Für alle anderen Stämme wurde das Wachstum erst nach einer verlängerten Inkubation über Nacht sichtbar.

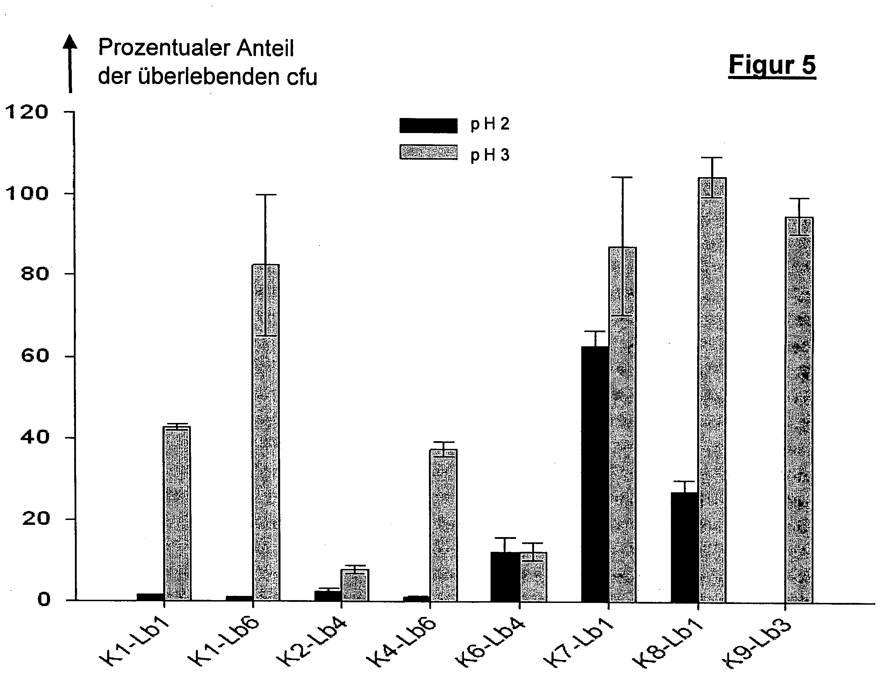

Wenn die Stämme drei Stunden pH-Werten von 2 und pH-Werten von 3 ausgesetzt waren, zeigten sie die verschiedene Stufen des Überlebens, die Figur 5 wiedergibt. Bei einem pH-Wert 3 zeigten vier Stämme, nämlich K1-Lb6, K7-Lb1, K8-Lb1 und K9-Lb3 Überlebensraten von mehr als 80%. Zwei von diesen, nämlich die Stämme K7-Lb1 und K8-Lb1 erwiesen die größte Belastbarkeit gegenüber den meisten Säuren mit Überlebensraten von 52,9% beim pH-Wert 2,0 und 27,2% beim pH-Wert von 6,5 im Verhältnis zu den Kontrollstämmen.

### 22. Cytokinproduktionsmuster

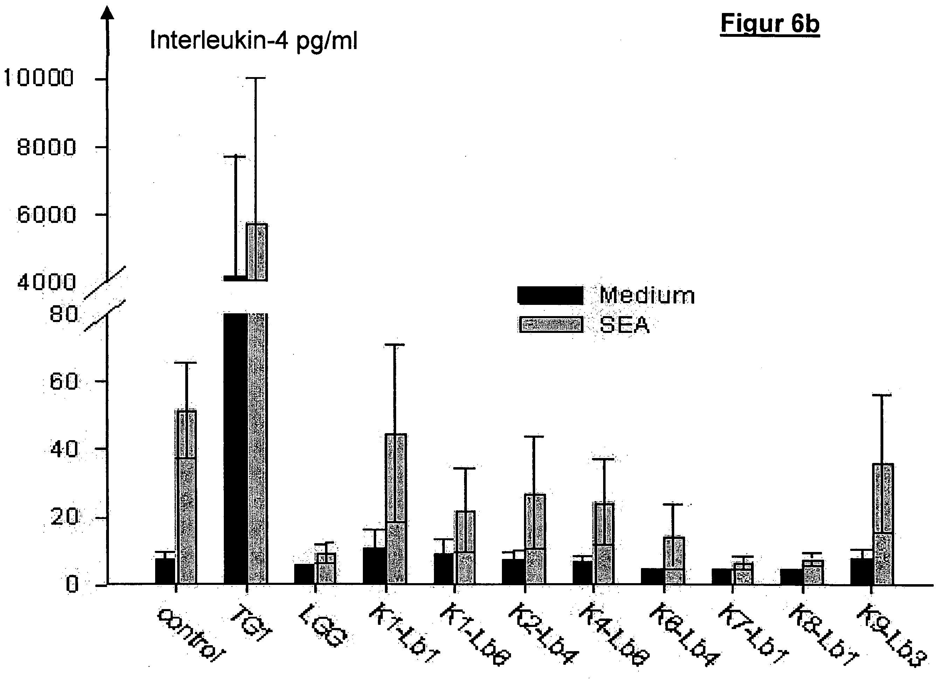

Die Kimerestämme unterdrückten die SEA-Induzierte Interferon-γ-Produktion, ein Phänomen, das für Stämme von Lactobacillus fermentum bislang nicht beobachtet worden ist. Die Kimerestämme unterdrückten die lnterleukin-4 Produktion. Bei den Stämmen K7-Lb1 und K8-Lb1 wurde das Interleukin-4 bis nahe an die Nachweisgrenze abgesenkt (Figur 6b).

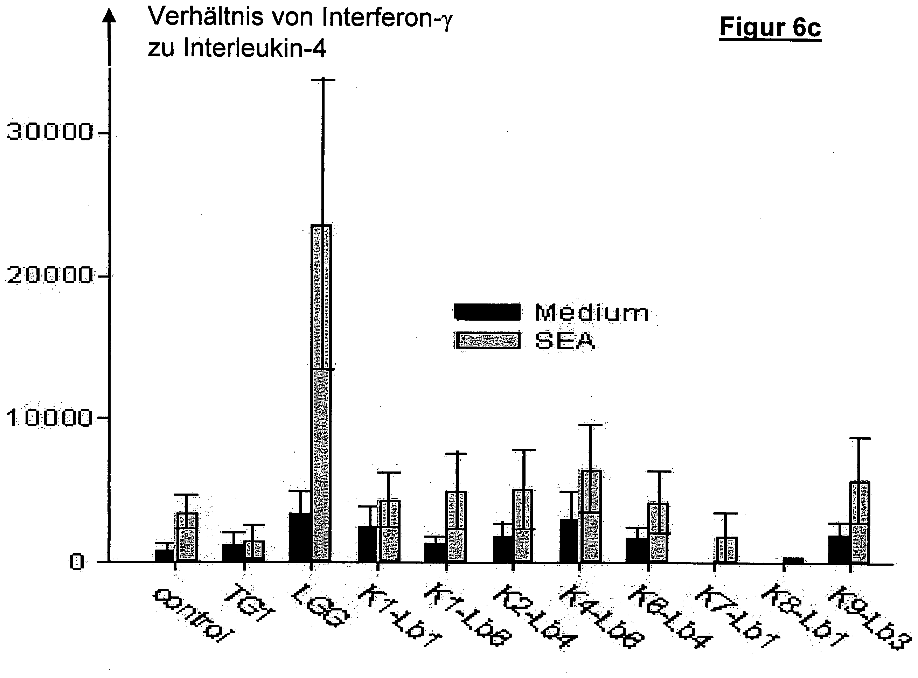

Eine vom jeweiligen Stamm abhängige Verschiebung des Interferony zu Interleukin-4-Verhältnisses und damit des dazu proportionalen T-Helfer1 zu T-Helfer2-Verhältnisses wurde beobachtet (Figur 6c).

Die Stämme K7-Lb1 und K8-Lb1 verschoben die T-Helfer1 zu T-Helfer2-Balance in Richtung auf T-Helfer2 , während die anderen einen Wechsel in Richtung auf T-Helfer1 induzierten. Alle geprüften Stämme waren im Vergleich mit TG1 - von dem die Unterdrückung der Interleukin-4 Produktion bekannt ist - in der Lage, die Produktion von Interleukin-4 signifikant zu unterdrücken. Verglichen mit den Kontrollstoffen unterdrückten die Stämme K6-Lb4 und K8-Lb1 die basale Sekretion von lnterleukin-4 (Figur 6b).

K7-Lb1 und K8-Lb1 unterdrückten die SEA-induzierte Interleukin-Absonderung (Figur 6b). Interleukin-4 reichte von 1,96 pg/ml für K8-Lb1 (fast nicht nachweisbar) bis zu 45 pg/ml für K1-Lb1 (Figur 6b).

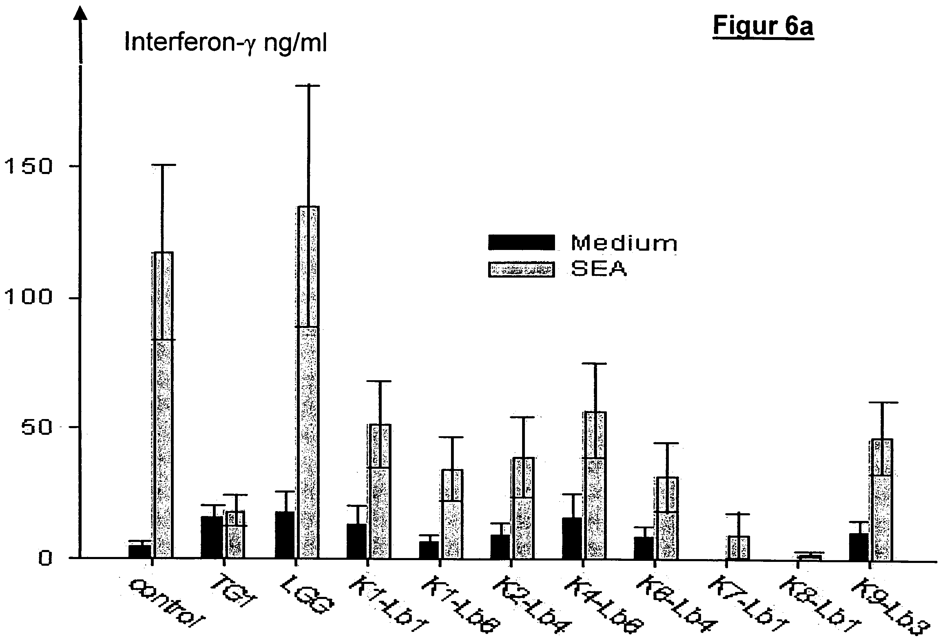

Die basale Interferon-γ-Produktion wurde signifikant unterdrückt durch die Stämme K7-Lb1 und K8-Lb1 (Figur 6a).

Eine Unterdrückung von SEA-induzierter Interferon-γ-produktion durch die getesteten Bakterienstämme konnte beobachtet werden. Sie war besonders prägnant für die Stämme K7-Lb1 und K8-Lb1, aber auch für alle anderen Kimerestämme (Figur 6a bis 6c).

Da die Überproduktion von Interferon-γ eine führende Rolle bei der Pathogenese von IBD spielt, zeigt die Verminderung von Interferon-γ durch die hier vorgestellten Stämme von Lactobacillus fermentum aus Kimere, dass diese Stämme eine immunmodulierende Eigenschaft haben, die im Kampf gegen Krankheiten genutzt werden kann, die von T-Helfer1-Zellen abhängig sind. Verschiedene Stämme von Lactobacillus fermentum aus Kimere induzierten entweder eine T-Helfer1 oder eine T-Helfer2 getriebene Antwort, abhängig von der jeweiligen Immunmodulation.

Die verschiedenen Stämme haben verschiedene Stimulationseffekte auf Interferon-γ und Interleukin-4 (Figur 6). Die Fähigkeit der Stämme, die basale Interleukin-4-Bildung zu unterdrücken, zeigt ihre mögliche Anwendung bei T-Helfer2-getriebenen Krankheiten.

Die Fähigkeit der Lactobacillus-fermentum-Isolate aus Kimere, die Bildung von SEA-Induziertem Interferon-γ zu unterdrücken und gleichzeitig die Produktion von lnterleukin-4 zu blockieren, zeigt einzigartige Möglichkeiten bei der Unterdrückung von Autoimmunkrankheiten und atopischen Krankheiten mit entzündlichen Komponenten.

Im Folgenden sollen weitere Einzelheiten und Merkmale der Erfindung anhand von graphischen Darstellungen erläutert werden. Diese sollen die Erfindung jedoch nicht einschränken, sondern nur erläutern. Es zeigt in schematischer Darstellung:
- Figur 1: Übersicht der Eigenschaften der einzelnen Stämme
- Figur 2: Stammbaum einiger hier vorgestellter Lactobazillen im Vergleich mit bereits bekannten Bakterien
- Figur 3: Bandenmuster aller 10 Stämme nach PFGE-Verfahren
- Figur 4: Toleranz der Stämme gegen Gallensalz
- Figur 5: Widerstandsfähigkeit der Stämme gegen sehr saure Umgebungen
- Figur 6: Th1- und die Th2-Cytokin-Produktion menschlicher Blutzellen (PBMC's) nach Ko-Incubation mit den Stämmen

In **Figur 1** sind als eine wesentliche Eigenschaft der hier vorgestellten Stämme charakteristische Wirkungen und deren Intensität dargestellt. Es wird deutlich, dass fast alle Stämme mit Ausnahme von K11-Lb3 jeweils drei charakteristische Eigenschaften zeigen, die sich voneinander unterscheiden. Alle dieser Stämme weisen eine mehr oder minder hohe Gallensalztoleranz und eine mehr oder mindere pH-Toleranz auf. Alle Stämme haben eine immunmodulierende Wirkung. Diese immunmodulierende Wirkung gehört entweder zur sog. natürlichen Immunität (natural oder innate immunity) wie die erhöhte Defensin-Freisetzung durch die Stämme hier K2-Lb6 und K11-Lb3 oder sie zählt zur adaptiven oder erworbenen Immunität, wie die Beeinflussung der Th1/Th2-Antwort.

Figur 1 zeigt, dass von den hier präsentierten Stämmen die K1-Lb1, K1-Lb6, K2-Lb4 und K4-Lb6 die Th1-Helferreaktion verstärken und den Th2-Einfluss reduziert. Die Stämme K2-Lb6, K6-Lb4, K7-Lb1 und K8-Lb1 hingegen verstärken die Th2-Antwort und reduzieren den Th1-Einfluss.

Die Intensität ist in drei Stufen wiedergegeben. Mit +++ ist der jeweils höchste der beobachteten Werte gekennzeichnet. Dementsprechend markiert ++ etwa 2/3 und + entspricht etwa 1/3.

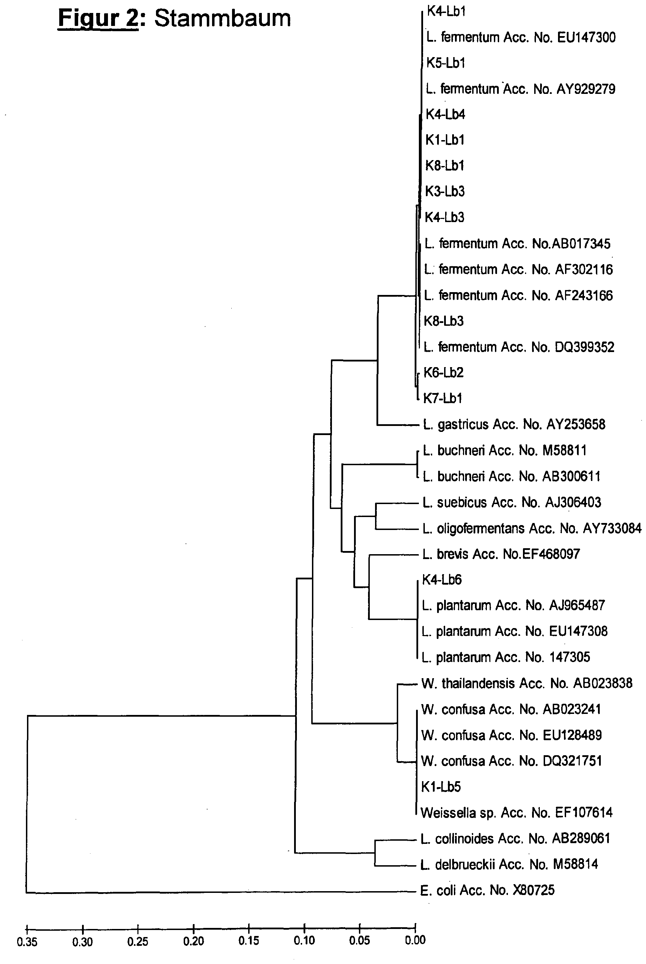

In **Figur 2** sind einige der aufgefundenen Stämme in einen Stammbaum eingetragen, in den auch einige andere, hinlänglich bekannte Bakterienstämme eingetragen sind. Dieser Stammbaum basiert auf partiellen Sequenzen der 16S rDNA von ausgewwählten Exemplaren der ARDRA-PCR Gruppierung von Isolaten der Lactobazillen aus Kimere verglichen mit der BLAST-Datenbasis. Die evolutionären Abstände wurden nach der UPMGA-Methode abgeleitet, wobei das Urladeprogramm auf 500 Wiederholungen basiert. Zur Berechnung der evolutionären Abstände wurde die Methode der maximalen zusammengesetzten Wahrscheinlichkeit benutzt. Diese phylogenetische Analyse wurde in MEGA4 gemäß Tamura et al 2007 ausgeführt.

In **Figur 3** sind die Bandenmuster aller 10 hier präsentierten Stämme nach dem PFGE-Verfahren, dem Pulsfeld-Gel-Elektrophoreseverfahren wiedergegeben. Es wird beim Betrachten von Figur 3 sofort deutlich, dass sich die einzelnen Bandenmuster deutlich voneinander unterscheiden. Alle Bakterien, die nach der PFGE-Methode analysiert werden und dabei genau dieses Bandenmuster zeigen, sind damit eindeutig als dieser Stamm der jeweiligen Art identifiziert.

Das Verfahren der Typisierung von Mikroorganismen durch PFGE ist seit 1984 bekannt und zählt zum anerkannten Stand der Technik. Es ermöglicht mit dem extrem geringen Aufwand der Hinterlegung einer einzigen Abbildung die eindeutige Identifikation des jeweiligen Bakterienstammes.

In den **Figuren 4a bis 4c** wird die Toleranz der Stämme gegen Gallensalz wiedergegeben. **Figur 4a** zeigt die Anzahl der überlebenden Stämme bei Exposition zu Rindergalle bei steigender Konzentration von 0,3% über 0,5%, 1 % und 2% bis zu 3%-iger Rindergallenkonzentration (w/v).

In **Figur 4b** wird durch die Messung der Absorption der Proben bei 620 nm das konkrete Wachstum der Stämme in 0,3%iger Rindergallenlösung über die Zeit hinweg dargestellt. Es zeigt sich für alle Stämme ein etwa ähnliches Verhalten.

**Figur 4c** zeigt die Ergebnisse einer prinzipiell gleichen Messung, jedoch mit 3,0%iger Konzentration der Rindergalle. Hier zeigt sich ein besonders schnelles Wachstum des ganz offensichtlich in Bezug auf Gallensalz sehr robusten Stammes K4-Lb6.

In **Figur 5** ist die Widerstandsfähigkeit der Stämme gegen sehr saure Umgebungen wiedergegeben und zwar für den pH-Wert 2 und den pH-Wert 3. Die Zellen wurden inkubiert in 0,35% NaCl (enthaltend 3gl-1 pepsin) und auf die pH-Werte 2, 3 und 6,5 für jeweils 3h bei 37°C eingestellt. Die überlebenden Zellen wurden auf einer MRS-Agarplatte bei 37°C für 48-72h gezählt. Die Werte bei pH 6,6 wurden als Referenzwert mit 100% definiert und die bei pH 2 und pH 3 Überlebenden Zellen mit diesem Referenzwert verglichen.

Die **Figuren 6a bis 6c** zeigen die Th1- und die Th2-Cytokin-Produktion von menschlichen Blutzellen (PBMC's) nach der Ko-Incubation mit Kimere-Lactobazillen-Stämmen und zwar mit und ohne Stimulation durch superantigenes Staphylococcus Enterotoxin A (SEA) in vitro. Als Wirkung der Bakterienstämme auf das Immunsystem und auf die Verschiebung des Th1/Th2-Verhältnisses wird in der Figur 6a die Produktion von Interferon-γ geziegt und in der Figur 6b die Produktion von lnterleukin-4 und in der Figur 6c das daraus entstehende Verhältnis von interferon-γ zu Interleukin-4.

## Patentansprüche

1. Pharmazeutische und/oder diätetische Zusammensetzung zur Verwendung zur Verstärkung der menschlichen Immunabwehr
**dadurch gekennzeichnet, dass** zur Verstärkung der adaptiven Immunabwehr mittels T-Helfer1- und T-Helfer2-Zellen Bakterien
- der Art Lactobacillus fermentum von wenigstens einem der Stämme
- K1-Lb1 oder
- K7-Lb1 oder
- K8 Lb1 oder
- der Art Lactobacillus plantarum vom Stamm K4-Lb6 und/oder zur Verstärkung der nativen Immunabwehr Bakterien der Art Lactobacillus fermentum von wenigstens einem der Stämme
- K2-Lb6 oder
- K11-Lb3
enthalten sind.

2. Zusammensetzung zur Verwendung nach dem vorhergehenden Anspruch, **dadurch gekennzeichnet, dass** die Verstärkung der adaptiven Immunabwehr eine Verschiebung von den T-Helfern2- zu den T-Helfern1-Reaktionen ist.

3. Zusammensetzung zur Verwendung nach Anspruch 2, **dadurch gekennzeichnet, dass** wenigstens einer der Stämme
- K1-Lb1 oder
- K4-Lb6
enthalten ist.

4. Zusammensetzung zur Verwendung nach Anspruch 1, **dadurch gekennzeichnet, dass** die Verstärkung der adaptiven Immunabwehr eine Verschiebung von den T-Helfern1-zu den- T-Helfern2-Antworten ist.

5. Zusammensetzung zur Verwendung nach Anspruch 2 oder 4, **dadurch gekennzeichnet, dass** die Verstärkung der Immunabwehr eine Hemmung von Entzündungen ist.

6. Zusammensetzung zur Verwendung nach Anspruch 4 oder 5, **dadurch gekennzeichnet, dass** wenigstens einer der Stämme
- K7-Lb1 oder
- K8-Lb1
enthalten ist.

7. Zusammensetzung zur Verwendung nach Anspruch 1, **dadurch gekennzeichnet, dass** wenigstens einer der Stämme
- K2-Lb6 oder
- K11-Lb3
enthalten ist und die Verstärkung der nativen Immunabwehr eine erhöhte Freisetzung von Defensin (hBD) aus Darmzellen ist.

8. Zusammensetzung zur Verwendung nach einem der vorhergehenden Ansprüche, **dadurch gekennzeichnet, dass** die Stämme eine 0,3%ige Gallensalzlösung tolerieren.

9. Zusammensetzung zur Verwendung nach einem der vorhergehenden Ansprüche, **dadurch gekennzeichnet, dass** der Stamm K4-Lb6 eine 3,0%ige Gallensalzlösung toleriert

10. Zusammensetzung zur Verwendung nach einem der vorhergehenden Ansprüche, **dadurch gekennzeichnet, dass** wenigstens 8% der Stämme einen pH-Wert von 3,0 für wenigstens 3h Überleben.

11. Zusammensetzung zur Verwendung nach Anspruch 10, **dadurch gekennzeichnet, dass** wenigstens einer der Stämme
- K1-Lb1 oder
- K2-Lb6 oder
- K4-Lb6 oder
- K7-Lb1 oder
- K8-Lb1
enthalten ist.

12. Zusammensetzung zur Verwendung nach einem der vorhergehenden Ansprüche, **dadurch gekennzeichnet, dass** zur Beeinflussung von Konzentration und Zusammensetzung glycosidischer Verbindungen an der Membran oder der Wand eines Bakteriums und damit zur Modulation der Immunantwort eines Wirts wenigstens einer der Stämme
- eine Glycosyltransferase oder
- eine Zuckertransferasen oder
- eine Acyltransferasen oder
- ein Lipoteichonsäure exportierendes Protein
exprimiert

13. Zusammensetzung zur Verwendung nach Anspruch 12, **dadurch gekennzeichnet, dass** bei wenigstens einem der Stämme zur Glycosyltransferase die Zuckerrest-Ankopplung durch die Diacylglycerol-Synthese (DAG) katalysierbar ist.

14. Zusammensetzung zur Verwendung nach Anspruch 12, **dadurch gekennzeichnet, dass** bei wenigstens einem der Stämme die Acyltransferase durch die Diacylglycerol-Synthese (DAG) katalysierbar ist.

15. Zusammensetzung zur Verwendung nach einem der vorhergehenden Ansprüche, **dadurch gekennzeichnet, dass** bei wenigstens einem der Stämme Enzyme weitere Zuckertransfers zur Synthese von mikrobiellen Peptidoglycanen katalysieren.

16. Zusammensetzung zur Verwendung nach einem der vorhergehenden Ansprüche, **dadurch gekennzeichnet, dass** bei wenigstens einem der Stämme
- durch eine Lipoteichonsäuresynthese und/oder
- durch eine Peptidoglycansynthese
als Transporter die Ausschleusung aus dem Cytoplasma vermittelbar ist.

17. Verwendung der Zusammensetzung nach den Ansprüchen 2 oder 3 zur
- Herstellung eines Arzneimittels oder
- eines Nahrungsmittels oder
- einer pharmazeutischen Zubereitung
zur prophylaktischen Behandlung oder zur Reduktion des Risikos der Manifestation oder zur Therapie von
- Ekzem und/oder
- atopischer Dermatitis und/oder
- Asthma und/oder
- Rhinitis allergica oder
- andere Allergien und/oder
- Tuberkulose und/oder
- Colitis ulcerosa und/oder
- Eosinophiler Pneumonie und/oder
- anderer Th2-getriebener Krankheiten oder Beschwerden

18. Verwendung der Zusammensetzung nach den Ansprüchen 2 oder 3 zur
- Herstellung eines Arzneimittels oder
- eines Nahrungsmittelzusatzes oder
- einer pharmazeutischen Zubereitung
zur prophylaktischen Behandlung oder zur Reduktion des Risikos der Manifestation oder zur Therapie von
- Darminfektionen und/oder
- Reisediarrhöen und/oder
- Erkältungen und/oder
- Urogenitalinfekte und/oder
- HIV-assoziierten Komplikationen und Beschwerden und/oder
- Candidiasis
oder anderen Beschwerden, Infekten oder anderen Krankheiten, deren Abwehr Th1-vermittelt ist.

19. Verwendung der Zusammensetzung nach den Ansprüchen 4 oder 6 zur
- Herstellung eines Arzneimittels oder
- eines Nahrungsmittelzusatzes oder
- einer pharmazeutischen Zubereitung
zur prophylaktischen Behandlung oder zur Reduzierung des Risikos der Manifestation oder zur Therapie von
- rheumatoider Arthritis und/oder
- Hashimoto Thyreoiditis und/oder
- Uveitis und/oder
- Psoriasis und/oder
- Typ1 Diabetes und/oder
- Morbus Sjögren und/oder
- Zoeliakie und/oder
- Systemischer Lupus erythematosus und/oder
- ankylosierender Spondylitis und/ oder
- Morbus Crohn und/oder
- entzündlichen Darmerkrankungen und/oder
- Sclerodera und/oder
- Sarkoidose und/oder
- Multiple Sklerose und/oder
- Vitiligo und/oder
- Basedow Autoimmunthyreoiditis und/oder
- endokriner Ophthalmopathie und/oder
- Myasthenia gravis und/oder
- Osteoarthritis und/oder
- Atherosklerose und in deren Folge von
- Herzinfarkt und/oder
- peripheren arteriellen Verschlusserkrankungen und/oder
- Hirninfarkt (Schlaganfall) und/oder
- metabolischem Syndrom und in deren Folge
- Adipositas und/oder
- Hypertonie und/oder
- Insulinresistenz und/oder
- Diabetes Typ2 und/oder
- Dyslipoproteinämie sowie - Reizdarmsyndrom (IBS) und/oder
- anderen T-Helfer1 getriebenen Beschwerden und Krankheiten.

20. Verwendung der Zusammensetzung nach den Ansprüchen 5 oder 4 zur
- Herstellung eines Arzneimittels oder
- eines Nahrungsmittelzusatzes oder
- einer pharmazeutischen Zubereitung
zur Reduktion oder Eliminierung der Auswirkungen
- von Autoimmunerkrankungen wie Arthritis oder Dermatiden oder
- von Allergien oder
- von Erkrankungen mit entzündlicher Komponente wie einem metabolischen Syndrom oder der in Anspruch 19 genannten Beschwerde und Krankheit mit entzündlicher Komponente.

21. Verwendung der Zusammensetzung nach einem der Ansprüche 9 bis 11 zur
- Herstellung eines Arzneimittels oder
- eines Nahrungsmittelzusatzes oder
- einer pharmazeutischen Zubereitung
zur Prophylaxe und Therapie von Osteoporose.

22. Verwendung der Zusammensetzung nach einem der Ansprüche 9 bis 11 zur
- Herstellung eines Arzneimittels oder
- eines Nahrungsmittelzusatzes oder
einer pharmazeutischen Zubereitung
zur unterstützenden Therapie bei hepatischer Encephalopathie durch geringere Resorption von Toxinen.

23. Verwendung der Zusammensetzung nach einem der Ansprüche 9 bis 11 zur
- Herstellung eines Arzneimittels oder
- eines Nahrungsmittelzusatzes oder
- einer pharmazeutischen Zubereitung
zur Reduktion der Beschwerden bei
- Reizdarmsyndrom.

24. Verwendung der Zusammensetzung nach einem der Ansprüche 9 bis 11 zur
- Herstellung eines Arzneimittels oder
- eines Nahrungsmittelzusatzes oder
- einer pharmazeutischen Zubereitung und/oder
zur Therapie von Vaginosis.

## Claims

1. Pharmaceutical and/or dietetic composition for use for strengthening the effect of the human immune defence, **characterised in that**, to strengthen the adaptive immune defence by means of T helper 1 and T helper 2 cells, bacteria
- of the genus Lactobacillus fermentum of at least one of the strains
- K1-Lb1 or
- K7-Lb1 or
- K8-Lb1 or
- of the genus Lactobacillus plantarum of the strain K4-Lb6 and/or, to strengthen the innate immune defence, bacteria of the genus Lactobacillus fermentum of at least one of the strains
- K2-Lb6 or
- K11-Lb3
are contained.

2. Composition for use according to the preceding claim, **characterised in that** the strengthening of the adaptive immune defence is a shift from the T helper 2 to the T helper 1 responses.

3. Composition for use according to claim 2, **characterised in that** at least one of the strains
- K1-Lb1 or
- K4-Lb6
is contained.

4. Composition for use according to claim 1, **characterised in that** the strengthening of the adaptive immune defence is a shift from the T helper 1 to the T helper 2 responses.

5. Composition for use according to claim 4, **characterised in that** the strengthening of the immune defence is an inhibition of inflammations.

6. Composition for use according to claim 4 or 5, **characterised in that** at least one of the strains
- K7-Lb1 or
- K8-Lb1
is contained.

7. Composition for use according to claim 1, **characterised in that** at least one of the strains
- K2-Lb6 or
- K11-Lb3
is contained and the strengthening of the innate immune defence is an increased release of defensin (hBD) from intestinal cells.

8. Composition for use according to one of the preceding claims, **characterised in that** the strains tolerate a 0.3% bile salt solution.

9. Composition for use according to one of the preceding claims, **characterised in that** the strain K4-Lb6 tolerates a 3.0% bile salt solution.

10. Composition for use according to one of the preceding claims, **characterised in that** at least 8% of the strains survive a pH of 3.0 for at least 3h.

11. Composition for use according to claim 10, **characterised in that** at least one of the strains
- K1-Lb1 or
- K2-Lb6 or
- K4-Lb6 or
- K7-Lb1 or
- K8-Lb1
is contained.

12. Composition for use according to one of the preceding claims, **characterised in that**, to influence the concentration and composition of glycosidic compounds at the membrane or at the wall of a bacterium, and therefore to modulate the immune response of a host, at least one of the strains expresses
- a glycosyl transferase or
- a sugar transferase or
- an acyl transferase or
- a lipoteichoic acid-exporting protein.

13. Composition for use according to claim 12, **characterised in that**, for at least one of the strains for glycosyl transferase, the sugar residue docking can be catalysed by diacylglycerol synthesis (DAG).

14. Composition for use according to claim 12, **characterised in that**, for at least one of the strains, the acyl transferase can be catalysed by diacylglycerol synthesis (DAG).

15. Composition for use according to one of the preceding claims, **characterised in that** for at least one of the strains, enzymes catalyse further sugar transfer for the synthesis of microbial peptidoglycans.

16. Composition for use according to one of the preceding claims, **characterised in that** in the case of at least one of the strains, the outward transfer from the cytoplasm can be mediated
- via a lipoteichoic acid synthesis and/or
- via a peptidoglycan synthesis
as transporter.

17. Use of the composition according to claim 2 or 3, for
- preparing a medication or
- a food supplement or
- a pharmaceutical preparation
for prophylactic treatment or reduction of the risk of manifestation or for therapy of
- eczema and/or
- atopic dermatitis and/or
- asthma and/or
- rhinitis allergica or
- other allergies and/or
- tuberculosis and/or
- colitis ulcerosa and/or
- eosinophilic pneumonia and/or
- other Th2-driven illnesses or complaints.

18. Use of the composition according to claim 2 or 3, for
- preparing a medication or
- a food supplement or
- a pharmaceutical preparation
- travel diarrhoea and/or
- colds and/or
- urogenital infections and/or
- HIV-associated complications and complains and/or
- candidiasis
or other complaints, infections or other illnesses the defence against which is Th1-mediated.

19. Use of the composition according to claims 4 to 6, for
- preparing a medication or
- a food supplement or
- a pharmaceutical preparation
for prophylactic treatment or reduction of the risk of manifestation or for therapy of
- rheumatoid arthritis and/or
- Hashimoto thyroiditis and/or
- uveitis and/or
- psoriasis and/or
- type 1 diabetes and/or
- Sjögren's syndrome and/or.
- coeliac disease and/or
- systemic lupus erythematosus and/or
- ankylosing spondylitis and/or
- Crohn's disease and/or
- inflammatory intestinal diseases and/or
- sclerodera and/or
- sarcoidosis and/or
- multiple sclerosis and/or
- vitiligo and/or
- Grave's autoimmune thyroiditis and/or
- endocrinous opthalmopathy and/or
- myasthenia gravis and/or
- osteoarthritis and/or
- arteriosclerosis and therefore also
- cardiac infarction and/or
- peripheral arterial occlusive diseases and/or
- cerebral infarction (stroke) and/or
- metabolic syndrome and therefore also
- adipositas and/or
- hypertonia and/or
- insulin resistance and/or
- type 2 diabetes and/or
- dyslipoproteinaemia as well as
- irritable bowel syndrome (IBS)
- other T helper 1-driven complains and illnesses.

20. Use of the composition according to claim 5 or 6, for
- preparing a medication or
- a food supplement or
- a pharmaceutical preparation
- for the reduction or elimination of the effects
- of autoimmune diseases such as arthritis or dermatitis or
- of allergies or
- of illnesses with inflammatory components such as a metabolic syndrome or arteriosclerosis or those complaints and illnesses with inflammatory component mentioned in claim 22.

21. Use of the composition according to one of claims 9 to 11, for
- preparing a medication or
- a food supplement or
- a pharmaceutical preparation
for prophylaxis and therapy of osteoporosis.

22. Use of the composition according to one of claims 9 to 11, for
- preparing a medication or
- a food supplement or
- a pharmaceutical preparation
for supportive therapy in the case of hepatic encephalopathy due to lower resorption of toxins.

23. Use of the composition according to one of claims 9 to 11, for
- preparing a medication or
- a food supplement or
- a pharmaceutical preparation
- for the reduction of complaints in the case of
- irritable bowel syndrome

24. Use of the composition according to one of claims 9 to 11, for
- preparing a medication or
- a food supplement or
- a pharmaceutical preparation and/or
for therapy of vaginosis.

## Revendications

1. Composition pharmaceutique et/ou diététique destinée à être utilisée afin de renforcer la défense immunitaire humaine,
***caractérisée par le fait qu'**elle* contient, afin de renforcer la défense immunitaire adaptative au moyen des lymphocytes T1 et T2 auxiliaires, des bactéries
- du type Lactobacillus fermentum d'au moins une des souches
- K1-Lb1 ou
- K7-Lb1 ou
- K8 Lb1 ou
- du type Lactobacillus plantarum de la souche K4-Lb6 et/ou, afin de renforcer la défense immunitaire innée, des bactéries du type Lactobacillus fermentum d'au moins une des souches
- K2-Lb6 ou
- K11-Lb3.

2. Composition destinée à être utilisée selon la revendication précédente, ***caractérisée par le fait que*** le renforcement de la défense immunitaire innée consiste en un déplacement des réactions des lymphocytes T1 auxiliaires vers celles des lymphocytes T2 auxiliaires.

3. Composition destinée à être utilisée selon la revendication 2, ***caractérisée par le fait qu'***au moins une des souches
- K1-Lb1 ou
- K4-Lb6
est contenue.

4. Composition destinée à être utilisée selon la revendication 1, ***caractérisée par le fait que*** le renforcement de la défense immunitaire adaptative consiste en un déplacement des réponses des lymphocytes T1 auxiliaires vers celles des lymphocytes T2 auxiliaires.

5. Composition destinée à être utilisée selon la revendication 2 ou 4, ***caractérisée par le fait que*** le renforcement de la défense immunitaire consiste en une inhibition d'inflammations.

6. Composition destinée à être utilisée selon la revendication 4 ou 5, ***caractérisée par le fait* qu'**au moins une des souches
- K7-Lb1 ou
- K8-Lb1
est contenue.

7. Composition destinée à être utilisée selon la revendication 1, ***caractérisée par le fait* qu'**au moins une des souches
- K2-Lb6 ou
- K11-Lb3
est contenue et que le renforcement de la défense immunitaire innée consiste en une libération accrue de défensine (hBD) provenant des cellules intestinales.

8. Composition destinée à être utilisée selon une des revendications précédentes, ***caractérisée par le fait que*** les souches tolèrent une solution de sel biliaire à 0,3 %.

9. Composition destinée à être utilisée selon une des revendications précédentes, ***caractérisée par le fait que*** la souche K4-Lb6 tolère une solution de sel biliaire à 0,3 %.

10. Composition destinée à être utilisée selon une des revendications précédentes, ***caractérisée par le fait*** q**u'**au moins 8% des souches survivent à un taux de pH de 3,0 pendant au moins 3h.

11. Composition destinée à être utilisée selon la revendication 10, ***caractérisée par le fait* qu'**au moins une des souches
- K1-Lb1 ou
- K2-Lb6 ou
- K4-Lb6 ou
- K7-Lb1 ou
- K8-Lb1
est contenue.

12. Composition destinée à être utilisée selon une des revendications précédentes, ***caractérisée par le fait que*** pour influencer la concentration et la composition de composés glycosidiques sur la membrane ou la paroi d'une bactérie et ainsi sur la modulation de la réponse immunitaire d'un hôte, au moins une des souches exprime une protéine exportant
- une glycosyltransférase ou
- une transférase de sucre ou
- une acyltransférase ou
- un acide lipotéichoïque.

13. Composition destinée à être utilisée selon la revendication 12, ***caractérisée par le fait que*** pour au moins une des souches destinée à la glycosyltransférase, le couplage de radical de sucre peut être catalysée par la synthèse de diacylglycérol (DAG).

14. Composition destinée à être utilisée selon la revendication 12, ***caractérisée par le fait* qu'**au moins une des souches peut catalyser l'acyltransférase par la synthèse de diacylglycérol (DAG).

15. Composition destinée à être utilisée selon une des revendications précédentes, ***caractérisée par le fait que*** pour au moins une des souches, des enzymes catalysent d'autres transferts de sucre pour la synthèse de peptidoglycanes microbiens.

16. Composition destinée à être utilisée selon une des revendications précédentes, ***caractérisée par le fait que*** pour au moins une des souches, l'exclusion hors du cytoplasme peut être fournie en tant que transporteur
- par une synthèse d'acide lipotéichoïque et/ou
- par une synthèse de peptidoglycane.

17. Utilisation de la composition d'après les revendications 2 ou 3 destinée à
- fabriquer un médicament ou
- un aliment ou
- une préparation pharmaceutique
pour traiter au plan prophylactique ou pour réduire le risque de la manifestation ou pour la thérapie
- d'eczéma et/ou
- de dermatite atopique et/ou
- d'asthme et/ou
- de rhinite allergique ou
- d'autres allergies et/ou
- de tuberculose et/ou
- de colite ulcéreuse et/ou
- de pneumonie éosinophile et/ou
- d'autres maladies ou pathologies stimulées par la Th2.

18. Utilisation de la composition d'après les revendications 2 ou 3 pour
- fabriquer un médicament ou
- un additif alimentaire ou
- une préparation pharmaceutique
pour traiter au plan prophylactique ou pour réduire le risque de la manifestation ou pour la thérapie
- d'infections intestinales et/ou
- de diarrhées de voyage et/ou
- des rhumes et/ou
- des infections urogénitales et/ou
- des complications et les pathologies liés au VIH et/ou
- de candidose
ou d'autres pathologies, infections ou autres maladies dont la défense est fournie par le Th1.

19. Utilisation de la composition d'après les revendications 4 ou 6 pour
- fabriquer un médicament ou
- un additif alimentaire ou
- une préparation pharmaceutique
pour traiter au plan prophylactique ou pour réduire le risque de la manifestation ou pour la thérapie
- de l'arthrite rhumatoïde et/ou
- de la thyroïdite de Hashimoto et/ou
- de l'uvéite et/ou
- du psoriasis et/ou
- du diabète de type 1 et/ou
- du syndrome de Sjögren et/ou
- de maladie coeliaque et/ou
- du lupus érythémateux disséminé et/ou
- de la spondylite ankylosante et/ ou
- de la maladie de Crohn et/ou
- de maladies intestinales inflammatoires et/ou
- de sclérose et/ou
- de sarcoïdose et/ou
- de sclérose multiple et/ou
- de vitiligo et/ou
- d'hyperthyroïdie auto-immune de Basedow et/ou
- d'ophtalmopathie endocrinienne et/ou
- de myasthénie grave et/ou
- d'ostéoarthrite et/ou
- d'athérosclérose et de ses conséquences et/ou
- d'infarctus du myocarde et/ou
- d'artériopathie oblitérante périphérique et/ou
- d'infarctus cérébral (attaque) et/ou
- de syndrome métabolique et de ses conséquences et/ou
- d'obésité et/ou
- d'hypertonie et/ou
- de résistance à l'insuline et/ou
- de diabète de type 2 et/ou
- de dyslipoprotéinémie ainsi que de syndrome du côlon irritable (SCI) et/ou
- d'autres pathologies et maladies favorisées par les T1 auxiliaires.

20. Utilisation de la composition d'après les revendications 5 ou 4 pour
- fabriquer un médicament ou
- un additif alimentaire ou
- une préparation pharmaceutique
destinés à réduire ou à éliminer les effets
- de maladies auto-immunes telles que l'arthrite ou les dermatites ou
- d'allergies ou
- de maladies comprenant des composantes inflammatoires comme un syndrome métabolique ou des pathologies et maladies nommées dans la revendication 19 comprenant des composantes inflammatoires.

21. Utilisation de la composition selon une des revendications 9 à 11 pour
- fabriquer un médicament ou
- un additif alimentaire ou
- une préparation pharmaceutique
destinés à la prophylaxie et à la thérapie de l'ostéoporose.

22. Utilisation de la composition selon une des revendications 9 à 11 pour
- fabriquer un médicament ou
- un additif alimentaire ou
- une préparation pharmaceutique
destinés à une thérapie de soutien en cas d'encéphalopathie hépatique due à une moindre résorption de toxines.

23. Utilisation de la composition selon une des revendications 9 à 11 pour
- fabriquer un médicament ou
- un additif alimentaire ou
- une préparation pharmaceutique
destinés à réduire les troubles ressentis en cas de
- syndrome du côlon irritable.

24. Utilisation de la composition selon une des revendications 9 à 11 pour
- fabriquer un médicament ou
- un additif alimentaire ou
- une préparation pharmaceutique
destinés à la thérapie de vaginose.
